# EUROPEAN PATENT APPLICATION

(11) **EP 4 700 042 A1**
(43) Date of publication of application: **25.02.2026**
(21) Application number: 24807534.3
(22) Date of filing: 14.05.2024
(51) Int. Cl.: C07K 14/34, C12N 15/77, C12P 19/32, C12R 1/15

(54) **MICROORGANISM PRODUCING PURINE NUCLEOTIDES AND METHOD FOR PRODUCING PURINE NUCLEOTIDES BY USING SAME**

(30) Priority: 16.05.2023 KR 20230063197
(71) Applicant: CJ Cheiljedang Corporation, Seoul 04560 (KR)
(72) Inventor: LEE, Ji Hyun, Seoul 04560 (KR); KIM, Eunji, Seoul 04560 (KR); KIM, Dae Young, Seoul 04560 (KR); BAE, Hyun-jung, Seoul 04560 (KR); CHOI, Jin-Geun, Seoul 04560 (KR); HUH, Lan, Seoul 04560 (KR)
(74) Representative: Meissner Bolte Partnerschaft mbB
(86) International application number: PCT/KR2024/006562
(87) International publication number: WO 2024/237663

(57) **Abstract**

Provided are a microorganism producing a purine nucleotide and a method of producing a purine nucleotide using the same.

## Description

### [Technical Field]

The present disclosure relates to a microorganism producing a purine nucleotide and a method of producing a purine nucleotide using the same.

### [Background Art]

5'-xanthosine monophosphate (XMP), which is an intermediate in the nucleic acid biosynthesis metabolic systems, is of physiological significance in the bodies of animals and plants, and is also applied in many fields for foods, pharmaceuticals, and various medical purposes. In particular, XMP is one of the nucleic acid-based seasonings that, like monosodium glutamate (MSG), has gained popularity as a flavor enhancer due to its synergistic effects when added to foods, and it is a precursor of 5'-guanosine monophosphate (GMP).

For the production of XMP, GMP, and other useful substances, various studies have been conducted to develop microorganisms with high-efficiency production and fermentation process technology. For example, target substance-specific approaches are mainly used, such as increasing the expression of genes encoding enzymes involved in the biosynthesis of XMP or GMP, or removing unnecessary genes in the biosynthesis (EP 3722430 A1, US 2020-0347346 A1).

However, as the demand for XMP or GMP increases, research is still needed to effectively increase the production capacity of XMP or GMP.

### [Disclosure]

### [Technical Problem]

The present inventors found that when a microorganism of the present disclosure is introduced with an MFS transporter variant, its purine nucleotide-producing ability is enhanced, as compared to the existing unmodified microorganism, thereby completing the present disclosure.

### [Technical Solution]

An aspect of the present disclosure provides wherein, in an amino acid sequence of SEQ ID NO: 1 or in an amino acid sequence having 75% or more sequence identity with SEQ ID NO: 1, any one amino acid selected from the group consisting of amino acids corresponding to positions 185, 238, 259, 283, 310, 354, 378, 383, and 403 of the amino acid sequence of SEQ ID NO: 1 is substituted with another amino acid.

In one specific embodiment, the substitution with another amino acid may be any one selected from the following substitutions:
(1) a substitution of the amino acid corresponding to position 259 with an amino acid selected from serine (S), threonine (T), phenylalanine (F), proline (P), methionine (M), glutamine (Q), and asparagine (N);
(2) a substitution of the amino acid corresponding to position 310 with an amino acid selected from alanine (A), phenylalanine (F), cysteine (C), isoleucine (I), glycine (G), methionine (M), tryptophan (W), serine (S), and threonine (T);
(3) a substitution of the amino acid corresponding to position 354 with an amino acid selected from serine (S), tyrosine (Y), asparagine (N), and leucine (L);
(4) a substitution of the amino acid corresponding to position 185 with glutamine (Q);
(5) a substitution of the amino acid corresponding to position 238 with threonine (T);
(6) a substitution of the amino acid corresponding to position 283 with glycine (G);
(7) a substitution of the amino acid corresponding to position 378 with alanine (A);
(8) a substitution of the amino acid corresponding to position 383 with leucine (L); and
(9) a substitution of the amino acid corresponding to position 403 with alanine (A).

In another specific embodiment, the variant may have a substitution of an amino acid corresponding to any one position selected from the group consisting of positions 231 and 347 in an amino acid sequence of SEQ ID NO: 3 with another amino acid.

In any one specific embodiment of the above specific embodiments, the variant may comprise a substitution selected from a substitution of the amino acid corresponding to positions 231 with threonine and a substitution of the amino acid corresponding to position 347 with serine in the amino acid sequence of SEQ ID NO: 3.

In another specific embodiment, the variant may have a substitution of an amino acid corresponding to any one position selected from the group consisting of positions 216 and 332 in an amino acid sequence of SEQ ID NO: 5 with another amino acid.

In any one specific embodiment of the above specific embodiments, the variant may comprise a substitution selected from a substitution of the amino acid corresponding to positions 216 with threonine and a substitution of the amino acid corresponding to positions 332 with serine in the amino acid sequence of SEQ ID NO: 5.

Another aspect of the present disclosure provides a polynucleotide encoding the MFS transporter variant.

Still another aspect of the present disclosure provides a vector comprising the polynucleotide.

Still another aspect of the present disclosure provides a microorganism of the genus *Corynebacterium,* the microorganism comprising any one or more selected from the MFS transporter variant; the polynucleotide encoding the MFS transporter variant; and the vector comprising the polynucleotide.

In one specific embodiment, the microorganism may have a purine nucleotide-producing ability.

In any one specific embodiment of the above specific embodiments, the microorganism may have an increased purine nucleotide-producing ability, as compared to a wild-type microorganism of the genus *Corynebacterium.*

In any one specific embodiment of the above specific embodiments, the purine nucleotide may be any one or more selected from XMP and GMP.

In another specific embodiment, the microorganism may be *Corynebacterium stationis.*

Still another aspect of the present disclosure provides a method of producing a purine nucleotide, the method comprising the step of culturing, in a medium, the microorganism of the genus *Corynebacterium* comprising any one or more selected from the MFS transporter variant; the polynucleotide encoding the MFS transporter variant; and the vector comprising the polynucleotide.

In one specific embodiment, the method of producing a purine nucleotide may further comprise the step of recovering the MFS transporter variant of the present disclosure which is expressed during the culturing step.

In another specific embodiment, the purine nucleotide may be any one or more selected from XMP and GMP.

Still another aspect of the present disclosure provides a composition for producing a purine nucleotide, the composition comprising the microorganism of the genus *Corynebacterium* comprising any one or more selected from the MFS transporter variant, the polynucleotide encoding the MFS transporter variant, and the vector comprising the polynucleotide; a culture medium thereof; or a combination thereof.

### [Advantageous Effects]

A microorganism of the present disclosure may have an increased purine nucleotide-producing ability by introducing an MFS transporter variant, as compared to the existing unmodified microorganism.

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below.

Further, those skilled in the art will recognize, or be able to ascertain using no more than routine experimentation, many equivalents to the specific embodiments of the disclosure described herein. Further, these equivalents should be interpreted to fall within the present disclosure.

Further, a number of papers and patent documents are referenced and cited throughout this specification. The disclosures of the cited papers and patent documents are incorporated herein by reference in their entirety to further clarify the level and scope of the subject matter to which the present disclosure pertains.

### Definition

As used in the specification and appended claims of the present disclosure, the singular forms ("a", "an", and "the") comprise plural referents unless the context clearly states otherwise. Unless the context indicates otherwise, singular terms shall comprise the plural and plural terms shall comprise the singular. As used in the specification and appended claims of the present disclosure, unless stated otherwise, the use of "or" may be used to comprise "and/or".

As used herein, the term "consisting essentially of" may mean that, in the case where the features of the object claimed herein are not substantially affected by the presence of an unspecified component, the unspecified component may be present.

As used herein, the term, "consisting of" means that the total ratio of specific component(s) is 100%. The components or features that are recited after the term "consisting of" may be essential or mandatory. In some embodiments, in addition to the components or features recited after the term "consisting of", any other components or non-essential components may be excluded.

As used herein, the term "comprising" means the presence of features, steps or components recited after the term, and does not exclude the presence or addition of one or more features, steps or components. The components or features recited after the term "comprising" herein may be essential or mandatory. However, in some embodiments, the term may further comprise any other or non-essential components or features.

### Variant

As used herein, the term "protein" or "polypeptide" refers to a polymer or oligomer of consecutive amino acid residues. In the present disclosure, "polypeptide," "protein," and "peptide" may be used interchangeably with "amino acid sequence."

In some cases, an amino acid sequence exhibiting activity may be referred to as an "enzyme". In the present disclosure, unless indicated otherwise, amino acid sequences are described in an N-terminal to C-terminal direction.

As used herein, the term "mature polypeptide" means a polypeptide in a form without a signal sequence or propeptide sequence. A mature protein/polypeptide/peptide may be a functional form of a protein/polypeptide/peptide. The mature polypeptide may be in a final form after translation or post-translational modification. Examples of the posttranslational modification comprise N- or C-terminal modifications, glycosylation, phosphorylation, leader sequence removal etc., but are not limited thereto.

As used herein, the term "wild-type" refers to a naturally-occurring state without artificial modification. When the term "wild-type" is used in reference to a polypeptide, it refers to a naturally occurring polypeptide, which does not have artificial mutations (substitutions, insertions, deletions, etc.) in one or more amino acid positions. Similarly, when the term "wild-type" is used in reference to a polynucleotide, it means not having artificial modifications (substitutions, insertions, deletions) in one or more nucleotides. However, polynucleotides encoding wild-type polypeptides are not limited to wild-type polynucleotides, and comprise sequences encoding any wild-type polypeptides.

As used herein, the parent sequence or backbone refers to a reference sequence in which modification is introduced to be a variant. That is, the parent sequence may be served as a starting sequence in which modification such as substitutions, additions, and/or deletions may be introduced. The parent sequence may be naturally-occurring or wild-type, or a variant in which one or more substitutions, insertions or deletions have occurred in the natural or wild type, or may be an artificially synthesized sequence. When the parent sequence is an amino acid sequence exhibiting activity, i.e., an amino acid sequence of an enzyme, it may be referred to as a parent enzyme.

As used herein, the term "reference sequence" means a sequence used to determine the position of amino acids within any amino acid sequence. Any amino acid sequence may be aligned with a reference sequence to determine the position of an amino acid that corresponds to a particular position in the reference sequence within any amino acid sequence.

As used herein, the term "position N" may comprise position N and an amino acid position corresponding to the position N, and specifically, may comprise an amino acid position corresponding to any amino acid residue in a mature polypeptide disclosed in a particular amino acid sequence.

As used herein, the term "corresponding to" refers to an amino acid residue at a position listed in a polypeptide, or an amino acid residue that is similar, identical or homologous to a residue listed in a polypeptide. Confirming the amino acid at the corresponding position may be determining a specific amino acid in a sequence that refers to a specific sequence. "Corresponding region" used herein generally refers to a similar or corresponding position in a related protein or reference protein.

In the present disclosure, SEQ ID NO: 1 may be used as a reference sequence to determine the position of an amino acid in any amino acid sequence.

In other words, SEQ ID NO: 1 disclosed in the present disclosure may be used to determine the corresponding amino acid residue in any polypeptide having the MFS transporter activity, and unless otherwise specified in the present disclosure, the residue of a specific amino acid sequence is numbered based on SEQ ID NO: 1.

For example, any amino acid sequence is aligned with SEQ ID NO: 1, and based on this, each amino acid residue of the amino acid sequence may be numbered by reference to the numerical position of the amino acid residue of SEQ ID NO: 1 and the corresponding amino acid residue. For example, a sequence alignment algorithm such as that described herein may confirm the position of an amino acid, or a position where a modification such as substitution, insertion or deletion occurs, compared to a query sequence (also referred to as a "reference sequence").

For such alignment, for example, Needleman-Wunsch algorithm (Needleman and Wunsch, 1970, J. Mol. Biol. 48: 443-453), Needle program of EMBOSS package (EMBOSS: The European Molecular Biology Open Software Suite, Rice et al., 2000), Trends Genet. 16: 276-277), and the like may be used without being limited thereto, and sequence alignment programs, pairwise sequence comparison algorithm, and the like known in the art may be appropriately used.

In addition, an amino acid residue corresponding to other polypeptide may be identified by multiple sequence alignment. Examples of the multiple sequence alignment program known in the art may comprise programs such as multiple sequence comparison by log-expectation (MUSCLE; version 3.5 or later; Edgar, 2004, Nucleic Acids Research 32: 1792-1797), MAFFT (version 6.857 or later; Katoh and Kuma, 2002, Nucleic Acids Research 30: 3059-3066; Katoh et al., 2005, Nucleic Acids Research 33: 511-518; Katoh and Toh, 2007, Bioinformatics 23: 372-374; Katoh et al., 2009, Methods in Molecular Biology 537: 39-64; Katoh and Toh, 2010, Bioinformatics 26: 1899-1900), etc., and EMBOSS EMMA using ClustalW (1.83 or later; Thompson et al., 1994, Nucleic Acids Research 22 : 4673-4680), etc., and respective default parameters thereof may be used, but are not limited thereto.

In addition, when relationship between polypeptides derived from the mature polypeptide cannot be detected by conventional sequence-based comparison, other pairwise sequence comparison algorithms may be used (Lindahl and Elofsson, 2000, J. Mol. Biol. 295: 613-615). Higher sensitivity in sequence-based searching may be obtained using search programs using probabilistic representations of polypeptide families (profiles) to search database. For example, PSI-BLAST program generates profiles through an iterative database search process and may detect remote homologs (Atschul et al., 1997, Nucleic Acids Res. 25: 3389-3402). Even higher sensitivity may be obtained when the family or superfamily for the polypeptide has one or more representatives in the protein structure database. Programs such as GenTHREADER (Jones, 1999, J. Mol. Biol. 287: 797-815; McGuffin and Jones, 2003, Bioinformatics 19: 874-881) utilize information from a variety of sources, such as PSI-BLAST, secondary structure prediction, structural alignment profiles, and solvation potentials, as input to a neural network that predicts the structural folding for a query sequence. Similarly, a method introduced by Gough et al., 2000, J. Mol. Biol. 313: 903-919 may be used to align a sequence of an unknown structure with the superfamily models present in the SCOP database. These alignments may be used in turn to generate a homology model for the polypeptide, and the models may be assessed for accuracy using a variety of tools developed for that purpose.

For proteins of known structures, several tools and resources are available for retrieving and generating structural alignment. For example, the SCOP superfamilies of proteins have been structurally aligned, and those alignments are accessible and downloadable. Two or more protein structures may be aligned using a variety of algorithms such as the distance alignment matrix (Holm and Sander, 1998, Proteins 33: 88-96) or Combinatorial extension (CE) Shindyalov and Bourne, 1998, Protein Engineering 11: 739-747). Implementation of these algorithms may additionally be utilized to query structure databases with a structure of interest in order to discover possible structural homologs (Holm and Park, 2000, Bioinformatics 16: 566-567).

The above-described methods are merely examples, and the present disclosure is not limited thereto.

With regard to an amino acid sequence in the present disclosure, although being described as a polypeptide "comprising" an amino acid sequence represented by a specific SEQ ID NO., a polypeptide "consisting of" an amino acid sequence represented by a specific SEQ ID NO., or a polypeptide or protein "having" an amino acid sequence represented by a specific SEQ ID NO., it is obvious that any protein having an amino acid sequence with deletion, modification, substitution, conservative substitution, or addition of some sequence may be comprised in the scope of the present disclosure as long as the protein has activity identical or corresponding to that of the protein consisting of the amino acid sequence of the corresponding SEQ ID NO. For example, as long as a protein has activity identical or corresponding to that of the variant protein, it does not exclude the addition of a sequence, which does not alter the function of the protein, upstream or downstream of the amino acid sequence, a naturally occurring mutation, a silent mutation thereof, or conservative substitution, and it is obvious that such a sequence addition or mutation also falls within the scope of the present disclosure.

For example, there are cases of having the addition of a sequence which does not alter the function of the variant polypeptide of the present disclosure at the N-terminus, C-terminus and/or within the amino acid sequence, a naturally occurring mutation, a silent mutation, or a conservative substitution.

As used herein, the term "conservative substitution" means substituting an amino acid with another amino acid having similar structural and/or chemical properties. Such amino acid substitution may generally occur based on similarity in the polarity, charge, solubility, hydrophobicity, hydrophilicity and/or amphipathic nature of the residue. Typically, the conservative substitution may have little or no effect on the activity of the polypeptide.

As used herein, the term "another amino acid" is not limited as long as it is an amino acid different from the amino acid before substitution. Meanwhile, when it is expressed that "a specific amino acid has been substituted", it is obvious that the amino acid is substituted with an amino acid different from the amino acid before substitution, even though it is not specifically stated that the amino acid has been substituted with a different amino acid.

In the present disclosure, the "varying/modifying" refers to changing or altering. This may be alteration from naturally occurring. For example, a polypeptide may be changed by altering the parent sequence or reference sequence of the polypeptide.

In the present disclosure, the modified polypeptide may be a polypeptide not existing in nature, i.e., a polypeptide which does not occur naturally.

As used herein, the term "modified" refers to, for example, altered from a naturally occurring form. A modified polypeptide of the present disclosure comprises a polypeptide which does not occur naturally or a naturally occurring variant. For example, the modified polypeptide of the present disclosure is a modified polypeptide not found in nature. For example, the modified polypeptide of the present disclosure may not be a spontaneously occurring polypeptide, but is not limited thereto.

When used in connection with an amino acid/nucleic acid sequence, the term "modification" used herein may comprise substitution of an amino acid/nucleic acid residue at one or more sites of a parent sequence with a different amino acid/nucleic acid residue, deletion of an amino acid/nucleic acid residue (or a series of amino acid/nucleic acid residues) of the parent sequence at one or more sites, insertion of an amino acid/nucleic acid residue (or a series of amino acid/nucleic acid residues) into the parent sequence at one or more sites, truncation of an amino acid sequence of the N-terminus and/or the C-terminus or a 5' and/or 3' nucleic acid sequence, and any combination thereof.

In the present disclosure, the "variant" or "variant polypeptide" refers to a polypeptide in which one or more amino acids are conservatively substituted and/or modified so as to be different from the polypeptide of the parent sequence, while retaining functions or properties of the polypeptide. The variant polypeptide differs from an identified sequence by substitution, deletion, or addition of several amino acids. Such a variant polypeptide may generally be identified by modifying one or more amino acids in the amino acid sequence of the polypeptide and evaluating the properties of the modified polypeptide. In other words, the ability of the variant polypeptide may be increased, unchanged, or decreased, compared to the native polypeptide. Further, some variant polypeptides may comprise variant polypeptides in which one or more portions, such as an N-terminal leader sequence or transmembrane domain, have been removed. Other variant polypeptides may comprise variants in which a portion has been removed from the N- and/or C-terminus of a mature protein. The term "variant polypeptide" may also be used as a modification, modified protein, mutant, mutein, divergent, variant, etc., and any term is not limited, as long as it is used in a sense of being mutated.

Further, the variant polypeptide may also comprise deletion or addition of amino acids that have minimal influence on the properties and secondary structure of the polypeptide. For example, a polypeptide may be conjugated to a signal (or leader) sequence at the N-terminus of the polypeptide, which co-translationally or post-translationally directs transfer of the protein. The polypeptide may also be conjugated to another sequence or a linker for identification, purification, or synthesis of the polypeptide.

The MFS transporter variant of the present disclosure may be in an isolated form.

As used herein, the term "isolated" refers to a substance present in an environment which does not occur naturally or present in a form which does not exist naturally. This comprises that a substance (sequence or nucleic acid) is at least substantially free from at least other components having the substance, e.g., sequence or nucleic acid, which is naturally associated in nature and as found in nature.

For example, the isolated sequence or nucleic acid provided in the present disclosure may be provided in a form that is substantially free of one or more contaminants.

Examples of the isolated substance may comprise i) any substance which is not naturally occurring, ii) any substance from which one or more, or all naturally-occurring components associated in nature are removed (e.g., enzyme, variant, nucleic acid, protein, peptide, or cofactor), iii) any substance that has been artificially modified from a substance found in nature, or iv) a substance modified to change the amount of the substance compared to other components naturally associated therewith (e.g., by increasing the copy number of a gene encoding a particular substance; by modifying a promoter naturally associated with a gene encoding a particular substance with a promoter having stronger activity, etc.), but are not limited thereto.

In the present discourse, in relation to an amino acid or nucleic acid sequence, the term "biologically active fragments or fragments" may refer to "functional fragments". The "functional fragment" may also refer to an active fragment, and refers to a polypeptide that comprises fewer amino acids than a full-length protein but possesses at least one biological activity of the corresponding full-length protein. For example, the functional fragment of the enzyme may comprise a catalytic site of the enzyme.

The MFS transporter variant of the present disclosure may comprise biologically active fragments of the MFS transporter variant. The biologically active fragments may comprise a part of the full length of a native polypeptide.

For example, the fragment may comprise at least about 20%, 30%, 40%, 50%, 60%, 70%, 80%, 90%, 95%, 98%, 99% or more, and less than 100% of amino acids of the full length of the native polypeptide, but is not limited thereto.

Throughout the specification, the conventional one-letter and three-letter codes for naturally occurring amino acids are used. In addition, the amino acids mentioned herein are abbreviated according to the nomenclature rules of IUPAC-IUB.

| | |
|---|---|
| alanine Ala, A | arginine Arg, R |
| asparagine Asn, N | aspartic acid Asp, D |
| cysteine Cys, C | glutamic acid Glu, E |
| glutamine Gln, Q | glycine Gly, G |
| histidine His, H | isoleucine Ile, I |
| leucine Leu, L | lysine Lys, K |
| methionine Met, M | phenylalanine Phe, F |
| proline Pro, P | serine Ser, S |
| threonine Thr, T | tryptophan Trp, W |
| tyrosine Tyr, Y | valine Val, V |

Meanwhile, any amino acid may be described as Xaa, X.

Also, three-letter codes generally allowed for other amino acids, such as 2-aminoisobutyric acid (Aib), N-methylglycine (Sar), and α-methyl-glutamic acid, may be used as well as the naturally occurring amino acids.

Amino acids may generally be classified based on similarity of polarity, charge, solubility, hydrophobicity, hydrophilicity, and/or amphipathic nature of residues.

Examples of this classification may comprise positively charged (basic) amino acids such as arginine, lysine, and histidine; negatively charged (acidic) amino acids such as glutamic acid and aspartic acid; amino acids with nonpolar side chains (nonpolar amino acids) such as glycine, alanine, valine, leucine, isoleucine, methionine, phenylalanine, tryptophan, and proline; amino acids with polar or hydrophilic side chains (polar amino acids) such as serine, threonine, cysteine, tyrosine, asparagine, and glutamine. For another example, amino acids may be classified into amino acids with electrically charged side chains (electrically charged amino acids) such as arginine, lysine, histidine, glutamic acid, and aspartic acid, and amino acids with uncharged side chains (uncharged amino acids; also called neutral amino acids) such as glycine, alanine, valine, leucine, isoleucine, methionine, phenylalanine, tryptophan, proline, serine, threonine, cysteine, tyrosine, asparagine, and glutamine. For still another example, phenylalanine, tryptophan, and tyrosine may be classified as aromatic amino acids. For still another example, valine, leucine, and isoleucine may be classified as branched amino acids. For still another example, 20 types of amino acids are classified according to size, starting from the amino acid group with relatively small volume, the amino acids may be classified into five groups; glycine, alanine, serine; cysteine, proline, threonine, aspartic acid, asparagine; valine, histidine, glutamic acid, glutamine; isoleucine, leucine, methionine, lysine, arginine; and phenylalanine, tryptophan, tyrosine. However, they are not necessarily limited thereto.

In order to describe the variant provided in the present disclosure, the following nomenclature is used.

In the present disclosure, referring to a specific position in an amino acid sequence may comprise referring to an amino acid present or substituted at that position. Referring to an amino acid at a specific position may be described in various ways. For example, the "position 003" may be described as "position 3", "amino acid 3", "3^{rd} amino acid". Further, for example, when the amino acid at position 3 is serine (S), it may be described as "S3" or "Ser3".

Amino acid substitution may be expressed by describing the amino acid before substitution, the position, and the amino acid to be substituted in sequence. The amino acids may be expressed using the conventional one-letter and three-letter codes. For example, when alanine, an amino acid at position 8 of a specific sequence, is substituted with valine, it may be described as "A8V" or "Ala8Val".

Any amino acid at a specific position may be referred to as "X". For example, X6 refers to any amino acid at position 6. In addition, when the amino acid to be substituted is expressed as X, it means that the amino acid is substituted with an amino acid different from the amino acid present before substitution. For example, "V6X" indicates that V at position 6 is substituted with any amino acid other than V.

### Polynucleotide

As used herein, the term "gene" means a polynucleotide that encodes a polypeptide and a polynucleotide comprising regions upstream and downstream of the coding region. In some embodiments, a gene may have a sequence (intron) inserted between respective coding regions (exons).

As used herein, the term "polynucleotide, nucleic acid, or nucleic acid molecule" refers to a DNA (e.g., cDNA or genomic DNA) or RNA strand of a certain length or longer as a polymer of nucleotides in which nucleotide monomers are connected in a long chain form by covalent bonds.

### Homology and Identity

As used herein, the term "homology" or "identity" refers to a degree of relevance between two given amino acid sequences or nucleotide sequences, and may be expressed as a percentage. The terms homology and identity may often be used interchangeably with each other.

The sequence homology or identity of a conserved polynucleotide or polypeptide is determined by standard alignment algorithms, and the default gap penalty established by a program used may be used together. Substantially, homologous or identical sequences may generally hybridize under moderately or highly stringent conditions to the entirety of the sequence or at least about 50%, 60%, 70%, 80%, or 90% of the full-length. It is apparent that hybridization also comprises hybridization of a polynucleotide with a polynucleotide comprising a general codon or a codon in consideration of codon degeneracy.

Whether or not any two polynucleotide or polypeptide sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined using Needleman-Wunsch algorithm (Needleman and Wunsch, 1970, J. Mol. Biol. 48: 443-453) as performed in the Needleman program of the EMBOSS package (EMBOSS: The European Molecular Biology Open Software Suite, Rice et al., 2000, Trends Genet. 16: 276-277) (version 5.0.0 or later) (comprising GCG program package ((Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO et al.](1988) SIAM J Applied Math 48: 1073). For example, BLAST of the National Center for Biotechnology Information or ClustalW may be used to determine the homology, similarity, or identity.

The homology, similarity, or identity between polynucleotides or polypeptides may be determined by comparing sequence information using the GAP computer program as introduced by, for example, Needleman et al. (1970), J Mol Biol. 48:443, as disclosed by Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of the symbols in the shorter of the two sequences. The default parameters for the GAP program may comprise: (1) unitary matrices (containing a value 1 for identity and a value 0 for non-identity), PAM Matrix (see those described by Schwartz and Dayhoff, eds., Atlas Of Protein Sequence And Structure, National Biomedical Research Foundation (1978)), and the weighted comparison matrix of Gribskov et al(1986) Nucl. Acids Res. 14: 6745 (or EDNAFULL (EMBOSS version of NCBI NUC4.4) substitution matrix); (2) a penalty of 3.0 for each gap and an additional 0.10 penalty for each symbol in each gap (or a gap open penalty of 10 and a gap extension penalty of 0.5); and (3) no penalty for end gaps.

Further, whether any two polynucleotide or polypeptide sequences have a homology, similarity or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual; F.M. Ausubel et al., Current Protocols in Molecular Biology, John Wiley & Sons, Inc., New York), but is not limited thereto.

As used herein, the term "stringent conditions" means conditions that enable specific hybridization between polynucleotides. These conditions are specifically described in documents (see Sambrook et al., supra, 9.50-9.51, 11.7-11.8). Examples thereof comprise a condition in which polynucleotides having higher homology or identity, i.e., polynucleotides having 60% or more, 70% or more, 75% or more, 80% or more, 85% or more, 90% or more, 95% or more, 96% or more, 97% or more, 98% or more, or 99% or more homology or identity are hybridized with each other while polynucleotides having lower homology or identity are not hybridized with each other, or a condition in which washing is performed once, specifically, twice to three times at a salt concentration and temperature equivalent to 60°C, 1XSSC, 0.1% SDS, specifically 60°C, 0.1XSSC, 0.1% SDS, more specifically, 68°C, 0.1XSSC, 0.1% SDS, which are washing conditions for common Southern hybridization.

The hybridization requires that two nucleotides have complementary sequences, although mismatches between bases are allowed depending on the stringency of hybridization. The term "complementary" is used to describe the relation between nucleotide bases capable of being hybridized with each other. For example, with regard to DNA, adenine is complementary to thymine and cytosine is complementary to guanine. Hence, the polynucleotide of the present disclosure may also comprise substantially similar nucleic acid sequences as well as isolated nucleic acid fragments that are complementary to the entire sequence.

The appropriate stringency to hybridize the polynucleotide depends on the length and degree of complementarity of the polynucleotide, and the variables are well known in the art (e.g., J. Sambrook et al., supra).

### Nucleic acid construct, Vector, Transformation

As used herein, the term "nucleic acid construct" refers to a single- or double-stranded nucleic acid molecule, which comprises one or more regulatory sequences, and which is artificially synthesized, or engineered to comprise a specific sequence in a manner that does not exist in nature, or isolated from nature.

As used herein, the term "vector" may comprise a DNA construct comprising a nucleotide sequence of a polynucleotide encoding a polypeptide of interest which is operably linked to a suitable expression regulatory region (or expression control sequence) so that the polypeptide of interest may be expressed in a suitable host. The expression regulatory region may comprise a promoter capable of initiating transcription, any operator sequence for controlling the transcription, a sequence encoding a suitable mRNA ribosome binding site, and a sequence controlling termination of transcription and translation. The vector may be transformed into a suitable microorganism, and then replicated or function independently of the host genome, or may be integrated into the genome itself.

The vector used in the present disclosure is not particularly limited, but any vector known in the art may be used. Examples of commonly used vectors comprise natural or recombinant plasmids, cosmids, viruses, and bacteriophages. For example, pWE15, M13, MBL3, MBL4, IXII, ASHII, APII, t10, t11, Charon4A, Charon21A, etc. may be used as a phage vector or a cosmid vector, and pDZ system, pDC system, pBR system, pUC system, pBluescript II system, pGEM system, pTZ system, pCL system, pET system, etc. may be used as a plasmid vector. For example, pCR, pDCM2, pDZ, pDC, pACYC177, pACYC184, pCL, pECCG117, pUC19, pBR322, pMW118, pCC1BAC vector, etc. may be used.

For example, a polynucleotide encoding a polypeptide of interest may be inserted into a chromosome through a vector for intracellular chromosome insertion. Insertion of the polynucleotide into the chromosome may be performed by any method known in the art, for example, homologous recombination, but is not limited thereto. The vector may further comprise a selection marker for the confirmation of chromosome insertion. The selection marker is for selecting the cells transformed with vectors, i.e., for confirming the insertion of a nucleic acid molecule of interest, and markers that confer selectable phenotypes such as drug resistance, auxotrophy, resistance to cytotoxic agents, or expression of surface polypeptides may be used. In an environment treated with a selective agent, only cells expressing the selection marker survive or exhibit other phenotypic traits, and thus transformed cells may be selected.

As used herein, the term "transformation" means that a vector comprising a polynucleotide encoding a target polypeptide is introduced into a host cell or a microorganism so that the polypeptide encoded by the polynucleotide may be expressed in the host cell. The transformed polynucleotide may be located regardless of the position, either by being inserted into the chromosome of the microorganism or located outside the chromosome as long as it may be expressed in the host cell. Further, the polynucleotide comprises DNA and RNA encoding a polypeptide of interest. The polynucleotide may be introduced in any form as long as it may be introduced into a host cell and expressed. For example, the polynucleotide may be introduced into a host cell in the form of an expression cassette, which is a gene construct containing all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

As used herein, the term "operably linked" refers to a constitution of placing a regulatory sequence to an appropriate position to direct expression of a coding sequence. Thus, the term "operably linked" comprises an attachment or linking between a regulatory region of a functional domain having a known or desired activity such as a promoter, a stop codon, a signal sequence, or an enhancer, and a target (gene or polypeptide) such that the expression, secretion, or function of the target may be regulated in accordance with the known or desired activity. For example, the term means that the polynucleotide sequence is functionally linked to a promoter sequence that initiates and mediates transcription of the polynucleotide encoding the desired variant polypeptide of the present disclosure.

As used herein, the term "expression" comprises any process involved in production of a polypeptide, e.g., transcription, post-transcriptional modification, translation, post-translational modification, and secretion, but is not limited thereto.

As used herein, the term "expression vector" refers to a linear or circular nucleic acid molecule comprising a coding sequence and an operably linked regulatory sequence for expression thereof.

As used herein, the term "regulatory sequence" refers to a polynucleotide sequence required for expression of a coding sequence. Each regulatory sequence may be native (from the same gene) or foreign (from different gene) to the coding sequence. Examples of the regulatory sequence may comprise a leader sequence, a polyadenylation sequence, a propeptide sequence, a promoter, a signal peptide sequence, an operator sequence, a ribosome binding site-encoding sequence, and a sequence terminating transcription and translation. A minimum unit of the regulatory sequence may comprise a promoter and a sequence terminating transcription and translation.

With regard to a cell, polynucleotide, polypeptide, or vector, the term "recombinant" used herein refers to the cell, polynucleotide, polypeptide, or vector modified by introduction of a heterologous nucleic acid or polypeptide or alteration of a native polynucleotide or polypeptide, or a cell derived from the modified cell. Thus, for example, a recombinant cell may express a gene that is not found within the native (non-recombinant) form of the cell or may express native genes which are either expressed or not expressed, or alternatively, abnormally expressed.

### Microorganism

As used herein, the term "microorganism (or strain)" comprises all of wild-type microorganisms or naturally or artificially genetically modified microorganisms, and it may be a microorganism in which a specific mechanism is weakened or strengthened due to insertion of a foreign gene or an activity enhancement or inactivation of an endogenous gene, and it may be a microorganism comprising genetic modification for production of a desired polypeptide, protein, or product. As used herein, the "microorganism" and "strain" have the same meaning and may be used interchangeably without limitation.

For example, the microorganism of the present disclosure may be a microorganism comprising the MFS transporter variant or a polynucleotide encoding the same; or a microorganism (e.g., recombinant strain) which is genetically modified to comprise the MFS transporter variant or the polynucleotide encoding the same.

The strain of the present disclosure may be a strain naturally having the purine nucleotide-producing ability or a microorganism obtained by providing the purine nucleotide-producing ability for a strain without the purine nucleotide-producing ability. For example, the strain may be a microorganism in which the purine nucleotide-producing ability is increased by introducing the MFS transporter variant of the present disclosure or the polynucleotide encoding the same, but is not limited thereto.

The strain of the present disclosure may be a microorganism having the increased purine nucleotide-producing ability, as compared to a parent strain without the variant of the present disclosure or a wild-type strain of the genus *Corynebacterium.* The microorganism may have the enhanced purine nucleotide-producing ability by introducing the MFS transporter variant of the present disclosure having the increased MFS transporter activity as compared to the wild-type MFS transporter or the polynucleotide encoding the MFS transporter variant. Specifically, the strain of the present disclosure may have the increased purine nucleotide-producing ability, as compared to a microorganism of the genus *Corynebacterium* comprising a wild-type MFS transporter having any one amino acid which has 75% or more sequence identity with SEQ ID NO: 1, or more specifically with SEQ ID NO: 1, 3, or 5 or a polynucleotide encoding the same.

As used herein, the term "microorganism having the purine nucleotide-producing ability" refers to a prokaryotic or eukaryotic microorganism capable of producing purine nucleotides within a living organism, and may comprise both a microorganism prepared by providing the purine nucleotide-producing ability for a parent strain without the purine nucleotide-producing ability, or a microorganism that inherently has the purine nucleotide-producing ability. The purine nucleotide-producing ability may be conferred or enhanced by species improvement.

As used herein, the term "unmodified microorganism" does not exclude strains comprising mutation that may occur naturally in microorganisms, and may be a wild-type strain or a natural strain itself or may be a strain before the trait is changed by genetic variation due to natural or artificial factors. The "unmodified microorganism" may be used interchangeably with "strain before being modified", "microorganism before being modified", "unvaried strain", "unmodified strain", "unvaried microorganism", "parent strain before being modified", "wild-type microorganism", "reference microorganism", or "standard microorganism". Further, as used herein, the term "MFS transporter unmodified microorganism" may refer to a strain into which the MFS transporter variant described herein is not introduced or has not yet been introduced. The MFS transporter unmodified microorganism of the present disclosure does not exclude a strain comprising modification of proteins or genes other than the modification of the MFS transporter or the polynucleotide encoding the same. Further, in the present disclosure, the unmodified microorganism may be a microorganism comprising any one amino acid which has 75% or more sequence identity with SEQ ID NO: 1, specifically sequence of SEQ ID NO: 1, 3, or 5 or any one polynucleotide of SEQ ID NO: 2, 4, or 6, but is not limited thereto.

### Increase of polypeptide activity

As used herein, the term "increase" in the activity of the polypeptide means that the activity of the polypeptide is enhanced, as compared with the endogenous activity. The increase may be used interchangeably with the term activation, up-regulation, overexpression, enhancement, etc.

The increase may comprise both cases in which an activity not originally possessed is exhibited, or the activity is enhanced compared to the endogenous activity or the activity before modification.

For example, "the activity not originally possessed is exhibited" may be "introduction of a protein", but is not limited thereto. The introduction of the protein means that a gene that is not originally possessed by a microorganism is expressed within the microorganism to exhibit the activity of a specific protein or to exhibit the increased or improved activity as compared to endogenous activity of the corresponding protein or the activity before modification. For example, a polynucleotide encoding a specific protein may be introduced into a chromosome in a microorganism, or a vector containing a polynucleotide encoding the specific protein may be introduced into the microorganism, thereby exhibiting its activity.

The "endogenous activity" means the activity of a specific polypeptide originally possessed by a parent strain before the trait is changed or an unmodified microorganism, when the trait is changed by genetic variation due to natural or artificial factors. This may be used interchangeably with "activity before modification".

The fact that the activity of a polypeptide is enhanced as compared to the endogenous activity means that the activity of the polypeptide is improved as compared to the activity and/or concentration (expression level) of the specific polypeptide originally possessed by a parent strain before the trait is changed or an unmodified microorganism.

For example, the increase indicates that the activity of the corresponding protein originally absent appears, or its activity or concentration is generally increased to about 1%, about 10%, about 25%, about 50%, about 75%, about 100%, about 150%, about 200%, about 300%, about 400% or about 500%, up to about 1000% or about 2000% or more, based on the activity or concentration in the wild-type protein or the initial microorganism strain, but is not limited thereto.

The increase of the activity of the polypeptide may be achieved through the introduction of a foreign polypeptide or the increase of the activity of the endogenous polypeptide. The increase of the activity of the polypeptide may be confirmed by increase in the degree of activity and the expression level of the corresponding polypeptide or increase in the amount of the product released from the corresponding polypeptide.

For the increase of the activity of the polypeptide, various methods well known in the art may be applied, and the method is not limited as long as the activity of the polypeptide of interest may be increased as compared to that of the microorganism before modification. Specifically, genetic engineering and/or protein engineering well known to those skilled in the art, which are routine methods of molecular biology, may be used, but the method is not limited thereto (e.g., Sitnicka et al. Functional Analysis of Genes. Advances in Cell Biology. 2010, Vol. 2. 1-16, Sambrook et al. Molecular Cloning 2012, etc.).

Specifically, the increase of the activity of the polypeptide of the present disclosure may be:
1) increase in the intracellular copy number of the polynucleotide encoding the polypeptide;
2) modification of a gene expression regulatory region on a chromosome encoding the polypeptide (e.g., occurrence of variations in the expression regulatory region, replacement with a sequence having stronger activity, or insertion of a sequence having stronger activity);
3) modification of a nucleotide sequence encoding an initiation codon or 5'-UTR region of the gene transcript encoding the polypeptide;
4) modification of the amino acid sequence of the polypeptide to increase the activity of the polypeptide;
5) modification of the polynucleotide sequence encoding the polypeptide to increase the activity of the polypeptide (e.g., modification of the polynucleotide sequence of the polypeptide gene to encode the polypeptide that has been modified to increase the activity of the polypeptide);
6) introduction of a foreign polypeptide exhibiting the activity of the polypeptide or a foreign polynucleotide encoding the same;
7) codon optimization of the polynucleotide encoding the polypeptide;
8) analysis of the tertiary structure of the polypeptide to select and to modify or chemically modify the exposed site; or
9) a combination of two or more selected from 1) to 8), but is not particularly limited thereto.

For example, 1) the increase in intracellular copy number of the polynucleotide encoding the polypeptide may be achieved by the introduction, into a host cell, of a vector to which the polynucleotide encoding the corresponding polypeptide is operably linked and which is able to replicate and function independently of the host. Alternatively, the increase may be achieved by the introduction of one or two or more copies of the polynucleotide encoding the corresponding polypeptide into the chromosome in a host cell. The introduction into the chromosome may be performed by the introduction, into a host cell, of a vector capable of inserting the polynucleotide into the chromosome in the host cell, but is not limited thereto. The vector is as described above.

2) The replacement of a gene expression control region (expression control sequence) on the chromosome encoding the polypeptide with a sequence with strong activity may be, for example, the mutation on the sequence through deletion, insertion, non-conservative or conservative substitution, or a combination thereof, or the replacement with a sequence having stronger activity, to further enhance the activity of the expression control region. The expression control region may comprise, but is not particularly limited to, a promoter, an operator sequence, a sequence encoding a ribosome binding site, a sequence controlling the termination of transcription and translation, etc. For example, it may be the replacement of the original promoter with a stronger promoter, but is not limited thereto.

Examples of the known stronger promoter may comprise cj1 to cj7 promoters (US Patent No. 7662943 B2), lac promoter, trp promoter, trc promoter, tac promoter, lamda phage PR promoter, PL promoter, tet promoter, gapA promoter, rhtB promoter, SPL7 promoter, SPL13 (sm3) promoter (US Patent No. 10584338 B2), O2 promoter (US Patent No. 10273491 B2), tkt promoter, yccA promoter, etc., but are not limited thereto.

3) The modification of a nucleotide sequence encoding an initiation codon or 5'-UTR region of the gene encoding the polypeptide may be, for example, the substitution with a nucleotide sequence encoding, rather than an endogenous initiation codon, another initiation codon having a higher expression rate of a polypeptide, but is not limited thereto.

4) and 5) The modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or the replacement with an amino acid sequence or polynucleotide sequence improved to have stronger activity or an amino acid sequence or polynucleotide sequence improved to have increased activity, in order to increase activity of the polypeptide, but is not limited thereto. Specifically, the replacement may be performed by inserting the polynucleotide into the chromosome by homologous recombination, but is not limited thereto. The vector used herein may further comprise a selection marker for checking the insertion of the chromosome. The selection marker is as described above.

6) The introduction of a foreign polypeptide exhibiting the activity of the polypeptide may be the introduction, into a host cell, of a foreign polynucleotide encoding a polypeptide exhibiting the same/similar activity with regard to the polypeptide. The foreign polynucleotide is not limited to the origin or sequence thereof as long as the foreign polynucleotide exhibits the same/similar activity with regard to the polypeptide. The method used in the introduction may be performed by any known transformation method appropriately selected by a person skilled in the art, and through the expression of the introduced polynucleotide in the host cell, the polypeptide is produced and the activity thereof may be increased.

7) The codon optimization of the polynucleotide encoding the polypeptide may be the codon optimization of an endogenous polynucleotide to increase transcription or translation thereof in a host cell, or the codon optimization of a foreign polynucleotide to allow optimized transcription or translation thereof in a host cell.

8) The modification or chemical modification of an exposed site selected by analysis of a tertiary structure of the polypeptide may be, for example, the modification or chemical modification of an exposed site to be modified or chemically modified by comparing sequence information of a polypeptide to be analyzed with a database that stores sequence information of known proteins, determining a template protein candidate according to similarity of the sequences, and identifying the structure based thereon.

Such increase of the activity of the polypeptide may mean that the activity or concentration of the corresponding polypeptide is increased relative to the activity or concentration of the polypeptide expressed in a wild-type or a microbial strain before modification, or that the amount of a product produced from the corresponding polypeptide is increased, but is not limited thereto.

The modification of a part or the entirety of the polynucleotide in the microorganism of the present disclosure may be induced by: (a) homologous recombination using a vector for chromosomal insertion in a microorganism or genome editing using engineered nuclease (e.g., CRISPR-Cas9), or and/or (b) the treatment with light, such as ultraviolet light and radiation, and/or chemicals, but is not limited thereto. The method of modifying a part or the entirety of the gene may comprise a method by DNA recombinant technology. For example, a nucleotide sequence or vector containing a nucleotide sequence homologous to a target gene may be introduced into the microorganism to bring about homologous recombination, resulting in deletion in a part or the entirety of the gene. The nucleotide sequence or vector to be introduced may comprise a dominant selection marker, but is not limited thereto.

### Culturing

As used herein, the term "culture" refers to growing the strain of the present disclosure in appropriately adjusted environment conditions. The culture procedure of the present disclosure may be performed according to appropriate media or culture conditions known in the art. Such culture may be easily adjusted according to the selected strain by a person skilled in the art. Specifically, the culture may be in a batch type, a continuous type, and/or a fed-batch type, but is not limited thereto.

As used herein, the "medium" refers to a mixture containing, as main ingredients, nutrient materials required for culturing the microorganism of the present disclosure, wherein the medium supplies nutrient materials comprising water essential for survival and growth, growth factors, etc. Specifically, as for the media and other culture conditions used for culturing the strain of the present disclosure, any medium that is used for usual culture of microorganisms may be used without particular limitation. However, the microorganism of the present disclosure may be cultured under aerobic conditions in a common medium containing appropriate carbon sources, nitrogen sources, phosphorus sources, inorganic compounds, amino acids, and/or vitamins, while controlling the temperature, pH, etc. For example, a culture medium for microorganisms of the genus *Corynebacterium* may be found in a literature ["Manual of Methods for General Bacteriology" by the American Society for Bacteriology (Washington D.C., USA, 1981)].

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates, such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphate sources may comprise potassium phosphate monobasic, potassium phosphate dibasic, and sodium-containing salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

The pH of the medium may be adjusted by adding compounds, such as ammonium hydroxide, potassium hydroxide, ammonia, phosphoric acid, and sulfuric acid, to the medium in an appropriate manner during the culture of the microorganism of the present disclosure. In addition, an anti-foaming agent, such as a fatty acid polyglycol ester, may be used to suppress foam formation during the culture. In addition, oxygen or oxygen-containing gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In the culture of the present disclosure, the culture temperature may be maintained at 20°C to 45°C, specifically, at 25°C to 40°C, and the culture may be performed for about 10 hours to 160 hours, but is not limited thereto.

As used herein, the term "culture" refers to a culture liquid obtained by culturing a specific microorganism in a culture medium, a concentrated culture liquid, a dry product of the culture liquid, a culture filtrate, a concentrated culture filtrate, or a dry product of the culture filtrate, and the culture liquid refers to those comprising the specific microorganism, and the culture filtrate refers to those substantially not comprising the specific microorganism (which means that the specific microorganism to be separated by filtration, etc. are substantially excluded, which does not mean that the microorganism is completely excluded from the filtrate.). The formulation of the culture is not limited, but it may be, for example, a liquid, an emulsion, or a solid.

As used herein, the term "fermentation" refers to a process in which microorganisms break down organic materials using their own enzymes, excluding putrefaction. Fermentation and putrefaction proceed by similar processes, but when useful substances are produced as a result of decomposition, it is called fermentation, and when bad smells or harmful substances are produced, it is called putrefaction.

In the present disclosure, the method of obtaining the fermentation product from the strain is not particularly limited, and the fermentation product may be obtained according to a method commonly used in the art or similar art.

As used herein, the term "fermentation product" comprises not only a fermented material itself, but also all types of materials comprising the fermentation product generated from the strain, such as a culture medium of a strain in which the strain and the culture coexist, a fermentation product obtained by filtering the strain from the culture medium, a fermentation product obtained by sterilizing the strain from the culture medium and filtering the same, an extract obtained by extracting the fermentation product or the culture medium comprising the same, a dilution obtained by diluting the fermentation product or the extract thereof, a concentrate, a dry product obtained by drying the fermentation product or the extract thereof, a lysate obtained by collecting and disrupting cells of the strain, etc.

### Detailed description of the present disclosure

Hereinafter, specific embodiments of the present disclosure will be described in more detail as follows.

An aspect of the present disclosure provides an MFS transporter variant.

As used herein, the term "MFS transporter" is one of the membrane transport proteins belonging to a major facilitator superfamily (MFS), and is a protein that promotes the movement of small solutes across the cell membrane in response to a chemiosmotic gradient. The MFS transporter may be a protein having the XMP-releasing activity. The term "MFS transporter" may be used interchangeably with "MFS transporter," "MFS carrier," "xmpE," or "xmpE protein".

The amino acid sequence of the MFS transporter may be available in the NCBI GenBank, etc., which is a known database.

The MFS transporter of the present disclosure may be derived from a microorganism of the genus of *Corynebacterium.*

For example, the wild-type of the MFS transporter provided in the present disclosure may be an MFS transporter derived from *Corynebacterium stationis, Corynebacterium J010B-136, Corynebacterium ammoniagenes, Corynebacterium case*i, *Corynebacterium glutamicum, Corynebacterium crudilactis,* or *Corynebacterium callunae.* Examples thereof may comprise a sequence of NCBI Accession number WP_088859234.1, HJG64956.1, WP_156489138.1, NME89867.1, HHT58321.1, WP_156845106.1, WP_105323823.1, AMJ45871.1, OAH32741.1, WP_205906775.1, WP_040355997.1, NMF31380.1, AQS74831.1, WP_205908328.1, HCJ69685.1, WP_006822858.1, WP_004568051.1, WP_011897033.1, WP_087061877.1, WP_077311687.1, WP_060564322.1, HJE11205.1, WP_065366770.1, WP_059289024.1, WP_038583410.1, WP_074491550.1, WP_011014092.1, WP_172768437.1, WP_087497622.1, WP_006285236.1, WP_096455209.1, WP_208400728.1, WP_208396345.1, NWO08766.1, WP_066565157.1, WP_003856921.1, WP_247776084.1 or WP_015650879.1, etc.

For another example, the wild-type MFS transporter may be a polypeptide comprising, consisting of, or essentially consisting of an amino acid sequence which has 60% or more, preferably 75% or more homology with SEQ ID NO: 1, specifically an amino acid sequence represented by any one of SEQ ID NO: 1, SEQ ID NO: 3, or SEQ ID NO: 5, but is not limited thereto.

In the present disclosure, the parent sequence of the MFS transporter, i.e., the MFS transporter subject to mutation, may comprise, without limitation, not only the wild-type MFS transporter described above, but also a sequence having the same activity as the amino acid sequence of the wild-type MFS transporter.

Specifically, the parent sequence of the MFS transporter may comprise an amino acid represented by any one of SEQ ID NO: 1, SEQ ID NO: 3, or SEQ ID NO: 5 or an amino acid having 60% or more, for example, at least 65%, 70%, 75%, 80%, 85%, 90%, 91%, 92%, 93%, 94%, 95%, 96%, 97%, 98% or more, or 99% or more homology or identity to any one of SEQ ID NO: 1, SEQ ID NO: 3, or SEQ ID NO: 5. Further, it is apparent that any protein having an amino acid sequence having deletion, modification, substitution, or addition in part of the sequence may also be comprised within the scope of the present disclosure as long as the amino acid sequence has such homology or identity and exhibits efficacy corresponding to that of the protein.

Meanwhile, in the present disclosure, when expressed as 'an amino acid corresponding to a specific position in a specific amino acid sequence is substituted', it is interpreted as being that the amino acid sequence is used as a reference sequence, wherein the parent sequence may have any structure unless otherwise defined. Examples of the parent sequence are as described above.

The MFS transporter variant provided in the present disclosure may be a variant having the MFS transporter activity, the variant comprising a substitution of an amino acid selected from the group consisting of amino acids corresponding to positions 185, 238, 259, 283, 310, 354, 378, 383, and 403 from the N-terminus of SEQ ID NO: 1 with another amino acid, in any polypeptide having the MFS transporter activity or the MFS transporter.

The MFS transporter variant of the present disclosure may have the enhanced MFS transporter activity as compared to the polypeptide before mutation, natural wild-type polypeptide, unmodified polypeptide, or polypeptide of the parent sequence.

Further, through sequence alignment, the positions corresponding to positions 238 and 354 from the N-terminus of SEQ ID NO: 1 may be positions 231 and 347 based on SEQ ID NO: 3, respectively and positions 216 and 332 based on SEQ ID NO: 5, respectively.

Therefore, one embodiment of the MFS transporter variant provided in the present disclosure may be an MFS transporter variant in which an amino acid corresponding to position selected from the group consisting of positions 231 and 347 of the amino acid sequence of SEQ ID NO: 3 is substituted with another amino acid, and/or an MFS transporter variant in which an amino acid corresponding to position selected from the group consisting of positions 216 and 332 of the amino acid sequence of SEQ ID NO: 5 is substituted with another amino acid.

Further, in the present disclosure, the description of the amino acid at position 238 of SEQ ID NO: 1 may also be applied to the amino acid at position 231 of SEQ ID NO: 3 and the amino acid at position 216 of SEQ ID NO: 5, and the description of the amino acid at position 354 of SEQ ID NO: 1 may also be applied to the amino acid at position 347 of SEQ ID NO: 3 and the amino acid at position 332 of SEQ ID NO: 5.

For example, the MFS transporter variant provided in the present disclosure may refer to a variant in which the amino acid corresponding to position 185 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, leucine (L) which is the amino acid corresponding to position 185 of the amino acid sequence of SEQ ID NO: 1 may be substituted with alanine, arginine, asparagine, aspartic acid, cysteine, glutamic acid, glutamine, glycine, histidine, isoleucine, lysine, methionine, phenylalanine, proline, serine, threonine, tryptophan, tyrosine, or valine.

For example, in the MFS transporter variant of the present disclosure, leucine which is the amino acid corresponding to position 185 of the amino acid sequence of SEQ ID NO: 1 may be substituted with glutamine.

For example, the variant, in which the amino acid corresponding to position 185 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be glutamine.

The MFS transporter variant may be a variant comprising, consisting of, or essentially consisting of an amino acid sequence represented by SEQ ID NO: 7, but is not limited thereto.

For another example, the MFS transporter variant provided in the present disclosure may refer to a variant in which the amino acid corresponding to position 238 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, isoleucine (I) which is the amino acid corresponding to position 238 of the amino acid sequence of SEQ ID NO: 1 may be substituted with alanine, arginine, asparagine, aspartic acid, cysteine, glutamic acid, glutamine, glycine, histidine, leucine, lysine, methionine, phenylalanine, proline, serine, threonine, tryptophan, tyrosine, or valine.

For example, in the MFS transporter variant of the present disclosure, isoleucine which is the amino acid corresponding to position 238 of the amino acid sequence of SEQ ID NO: 1 may be substituted with threonine.

For example, the variant, in which the amino acid corresponding to position 238 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be threonine.

Further, it is obvious that the MFS transporter variant of the present disclosure may comprise deletions or additions of amino acids that have minimal effect on the properties and secondary structure of the MFS transporter in which the amino acid corresponding to position 238 of SEQ ID NO: 1 is substituted with another amino acid as described. In addition, through sequence alignment known in the art, those skilled in the art may recognize that position 238 from the N-terminus of SEQ ID NO: 1 of the present disclosure corresponds to position 231 of SEQ ID NO: 3 and position 216 of SEQ ID NO: 5.

Accordingly, the MFS transporter variant of the present disclosure may be exemplified by a variant in which the amino acid corresponding to position 238 of SEQ ID NO: 1 (the amino acid at position 231 of SEQ ID NO: 3 and the amino acid at position 216 of SEQ ID NO: 5) is substituted, in connection with SEQ ID NO: 3 and SEQ ID NO: 5.

Further, in the present disclosure, the description of the amino acid at position 238 of SEQ ID NO: 1 may also be applied to the amino acid at position 231 of SEQ ID NO: 3 and the amino acid at position 216 of SEQ ID NO: 5.

For example, the MFS transporter variant of the present disclosure may comprise an amino acid sequence in which the amino acid corresponding to position 238 of SEQ ID NO: 1 is substituted with another amino acid and may have at least 75% or more and less than 100% sequence homology to SEQ ID NO: 1. In another embodiment, the MFS transporter variant of the present disclosure may comprise an amino acid sequence in which the amino acid corresponding to position 231 of SEQ ID NO: 3 is substituted with another amino acid and may have 75% or more and less than 100% sequence homology to SEQ ID NO: 3. In still another embodiment, the MFS transporter variant of the present disclosure may comprise an amino acid sequence in which the amino acid corresponding to position 216 of SEQ ID NO: 5 is substituted with another amino acid and may have 75% or more and less than 100% sequence homology to SEQ ID NO: 5.

The MFS transporter variant may be a variant comprising, consisting of, or essentially consisting of an amino acid sequence represented by any one of SEQ ID NO: 131, 133, and 137, but is not limited thereto.

For another example, the MFS transporter variant provided in the present disclosure may refer to a variant in which the amino acid corresponding to position 259 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, isoleucine (I) which is the amino acid corresponding to position 259 of the amino acid sequence of SEQ ID NO: 1 may be substituted with alanine, arginine, asparagine, aspartic acid, cysteine, glutamic acid, glutamine, glycine, histidine, leucine, lysine, methionine, phenylalanine, proline, serine, threonine, tryptophan, tyrosine, or valine.

For example, in the MFS transporter variant of the present disclosure, isoleucine which is the amino acid corresponding to position 259 of the amino acid sequence of SEQ ID NO: 1 may be substituted with an amino acid selected from serine, threonine, phenylalanine, proline, methionine, glutamine and asparagine.

For example, the variant, in which the amino acid corresponding to position 259 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be an amino acid selected from serine, threonine, phenylalanine, proline, methionine, glutamine and asparagine.

The MFS transporter variant may be a variant comprising, consisting of, or essentially consisting of an amino acid sequence represented by any one of SEQ ID NOS: 55, 57, 59, 75, 81, 85, and 91, but is not limited thereto.

For another example, the MFS transporter variant provided in the present disclosure may refer to a variant in which the amino acid corresponding to position 283 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, serine (S) which is the amino acid corresponding to position 283 of the amino acid sequence of SEQ ID NO: 1 may be substituted with alanine, arginine, asparagine, aspartic acid, cysteine, glutamic acid, glutamine, glycine, histidine, isoleucine, leucine, lysine, methionine, phenylalanine, proline, threonine, tryptophan, tyrosine, or valine.

For example, in the MFS transporter variant of the present disclosure, serine which is the amino acid corresponding to position 283 of the amino acid sequence of SEQ ID NO: 1 may be substituted with glycine.

For example, the variant, in which the amino acid corresponding to position 283 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be glycine.

The MFS transporter variant may be a variant comprising, consisting of, or essentially consisting of an amino acid sequence represented by SEQ ID NO: 15, but is not limited thereto.

For another example, the MFS transporter variant provided in the present disclosure may refer to a variant in which the amino acid corresponding to position 310 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, valine (V) which is the amino acid corresponding to position 310 of the amino acid sequence of SEQ ID NO: 1 may be substituted with alanine, arginine, asparagine, aspartic acid, cysteine, glutamic acid, glutamine, glycine, histidine, isoleucine, leucine, lysine, methionine, phenylalanine, proline, serine, threonine, tryptophan, or tyrosine.

For example, in the MFS transporter variant of the present disclosure, valine which is the amino acid corresponding to position 310 of the amino acid sequence of SEQ ID NO: 1 may be substituted with an amino acid selected from alanine, phenylalanine, cysteine, isoleucine, glycine, methionine, tryptophan, serine, and threonine.

For example, the variant, in which the amino acid corresponding to position 310 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be an amino acid selected from alanine, phenylalanine, cysteine, isoleucine, glycine, methionine, tryptophan, serine, and threonine.

The MFS transporter variant may be a variant comprising, consisting of, or essentially consisting of an amino acid sequence represented by any one of SEQ ID NO: 93, 95, 97, 105, 111, 117, 119, 121, and 123, but is not limited thereto.

For another example, the MFS transporter variant provided in the present disclosure may refer to a variant in which the amino acid corresponding to position 354 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, phenylalanine (F) which is the amino acid corresponding to position 354 of the amino acid sequence of SEQ ID NO: 1 may be substituted with alanine, arginine, asparagine, aspartic acid, cysteine, glutamic acid, glutamine, glycine, histidine, isoleucine, leucine, lysine, methionine, proline, serine, threonine, tryptophan, tyrosine, or valine.

For example, in the MFS transporter variant of the present disclosure, valine which is the amino acid corresponding to position 354 of the amino acid sequence of SEQ ID NO: 1 may be substituted with an amino acid selected from serine, tyrosine, asparagine, and leucine.

For example, the variant, in which the amino acid corresponding to position 354 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be an amino acid selected from serine, tyrosine, asparagine, and leucine.

Further, it is obvious that the MFS transporter variant of the present disclosure may comprise deletions or additions of amino acids that have minimal effect on the properties and secondary structure of the MFS transporter in which the amino acid corresponding to position 354 of SEQ ID NO: 1 is substituted with another amino acid as described. In addition, through sequence alignment known in the art, those skilled in the art may recognize that position 354 from the N-terminus of SEQ ID NO: 1 of the present disclosure corresponds to position 347 of SEQ ID NO: 3 and position 332 of SEQ ID NO: 5.

Accordingly, the MFS transporter variant of the present disclosure may be exemplified by a variant in which the amino acid corresponding to position 354 of SEQ ID NO: 1 (the amino acid at position 347 of SEQ ID NO: 3 and the amino acid at position 332 of SEQ ID NO: 5) is substituted, in connection with SEQ ID NO: 3 and SEQ ID NO: 5.

Further, in the present disclosure, the description of the amino acid at position 354 of SEQ ID NO: 1 may also be applied to the amino acid at position 347 of SEQ ID NO: 3 and the amino acid at position 332 of SEQ ID NO: 5.

For example, the MFS transporter variant of the present disclosure may comprise an amino acid sequence in which the amino acid corresponding to position 354 of SEQ ID NO: 1 is substituted with another amino acid and may have at least 75% or more and less than 100% sequence homology to SEQ ID NO: 1. In another embodiment, the MFS transporter variant of the present disclosure may comprise an amino acid sequence in which the amino acid corresponding to position 347 of SEQ ID NO: 3 is substituted with another amino acid and may have 75% or more and less than 100% sequence homology to SEQ ID NO: 3. In still another embodiment, the MFS transporter variant of the present disclosure may comprise an amino acid sequence in which the amino acid corresponding to position 332 of SEQ ID NO: 5 is substituted with another amino acid and may have 75% or more and less than 100% sequence homology to SEQ ID NO: 5.

The MFS transporter variant may be a variant comprising, consisting of, or essentially consisting of an amino acid sequence represented by any one of SEQ ID NO: 17, 19, 21, 51, 135, and 139, but is not limited thereto.

For another example, the MFS transporter variant provided in the present disclosure may refer to a variant in which the amino acid corresponding to position 378 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, valine (V) which is the amino acid corresponding to position 378 of the amino acid sequence of SEQ ID NO: 1 may be substituted with alanine, arginine, asparagine, aspartic acid, cysteine, glutamic acid, glutamine, glycine, histidine, isoleucine, leucine, lysine, methionine, phenylalanine, proline, serine, threonine, tryptophan, or tyrosine.

For example, in the MFS transporter variant of the present disclosure, valine which is the amino acid corresponding to position 378 of the amino acid sequence of SEQ ID NO: 1 may be substituted with alanine.

For example, the variant, in which the amino acid corresponding to position 378 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be alanine.

The MFS transporter variant may be a variant comprising, consisting of, or essentially consisting of an amino acid sequence represented by SEQ ID NO: 9, but is not limited thereto.

For another example, the MFS transporter variant provided in the present disclosure may refer to a variant in which the amino acid corresponding to position 383 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, isoleucine (I) which is the amino acid corresponding to position 383 of the amino acid sequence of SEQ ID NO: 1 may be substituted with alanine, arginine, asparagine, aspartic acid, cysteine, glutamic acid, glutamine, glycine, histidine, leucine, lysine, methionine, phenylalanine, proline, serine, threonine, tryptophan, tyrosine, or valine.

For example, in the MFS transporter variant of the present disclosure, isoleucine which is the amino acid corresponding to position 383 of the amino acid sequence of SEQ ID NO: 1 may be substituted with leucine.

For example, the variant, in which the amino acid corresponding to position 383 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be leucine.

The MFS transporter variant may be a variant comprising, consisting of, or essentially consisting of an amino acid sequence represented by SEQ ID NO: 11, but is not limited thereto.

For another example, the MFS transporter variant provided in the present disclosure may refer to a variant in which the amino acid corresponding to position 403 of SEQ ID NO: 1 is substituted with another amino acid as compared to the parent sequence, thereby having activity more than 100% of the protein of the parent sequence, among the proteins having the MFS transporter activity described above.

For example, in the MFS transporter variant of the present disclosure, valine (V) which is the amino acid corresponding to position 403 of the amino acid sequence of SEQ ID NO: 1 may be substituted with alanine, arginine, asparagine, aspartic acid, cysteine, glutamic acid, glutamine, glycine, histidine, isoleucine, leucine, lysine, methionine, phenylalanine, proline, serine, threonine, tryptophan, or tyrosine.

For example, in the MFS transporter variant of the present disclosure, valine which is the amino acid corresponding to position 403 of the amino acid sequence of SEQ ID NO: 1 may be substituted with alanine.

For example, the variant, in which the amino acid corresponding to position 403 from the N-terminus of SEQ ID NO: 1 is substituted with another amino acid, may comprise an amino acid sequence having at least 60%, 65%, 70%, 75%, 80%, 85%, 90%, 91%, 92%, 93%, 94%, 95%, 96%, 97%, 98%, or 99% or more homology or identity to SEQ ID NO: 1. For example, another amino acid may be alanine.

The MFS transporter variant may be a variant comprising, consisting of, or essentially consisting of an amino acid sequence represented by SEQ ID NO: 13, but is not limited thereto.

In one embodiment of the above-described embodiments, the MFS transporter variant provided in the present disclosure may be a variant having the MFS transporter activity, in which an amino acid corresponding to position selected from the group consisting of positions 231 and 347 from the N-terminus of SEQ ID NO: 3 is substituted with another amino acid, in any polypeptide having the MFS transporter activity or the MFS transporter. For example, the variant may comprise a substitution selected from a substitution of the amino acid corresponding to position 231 with threonine and a substitution of the amino acid corresponding to position 347 with serine, in the amino acid sequence of SEQ ID NO: 3.

In another embodiment of the above-described embodiments, the MFS transporter variant provided in the present disclosure may be a variant having the MFS transporter activity, in which an amino acid corresponding to position selected from the group consisting of positions 216 and 332 from the N-terminus of SEQ ID NO: 5 is substituted with another amino acid, in any polypeptide having the MFS transporter activity or the MFS transporter. For example, the variant may comprise a substitution selected from a substitution of the amino acid corresponding to position 216 with threonine and a substitution of the amino acid corresponding to position 332 with serine, in the amino acid sequence of SEQ ID NO: 5.

The MFS transporter variant may be a polypeptide comprising, consisting of, or essentially consisting of any one of an amino acid sequence selected from the group consisting of SEQ ID NO: 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, and 139; or a polypeptide having 60% or more, for example, 75% or more homology or sequence identity therewith, but is not limited thereto.

Another aspect of the present disclosure provides a polynucleotide encoding the MFS transporter variant of the present disclosure.

The polynucleotide encoding the MFS transporter variant of the present disclosure may comprise any polynucleotide sequence without limitation, as long as it encodes the MFS transporter variant having the enhanced activity of the present disclosure.

The polynucleotide of the present disclosure may undergo various modifications in the coding region without changing the amino acid sequence of the polypeptide, due to codon degeneracy or in consideration of the codons preferred in an organism in which the polypeptide is to be expressed. Specifically, the polynucleotide may comprise any polynucleotide sequence without limitation, as long as it is a polynucleotide sequence encoding a variant in which an amino acid selected from the group consisting of amino acids corresponding to positions 185, 238, 259, 283, 310, 354, 378, 383, and 403 from the N-terminus of SEQ ID NO: 1 is substituted with another amino acid.

For example, the polynucleotide of the present disclosure may be a polynucleotide sequence encoding the variant of the present disclosure, specifically, a polypeptide comprising, consisting of, or essentially consisting of an amino acid sequence represented by any one selected from SEQ ID NOS: 7, 9, 11, 13, 15, 17, 19, 21, 23, 25, 27, 29, 31, 33, 35, 37, 39, 41, 43, 45, 47, 49, 51, 53, 55, 57, 59, 61, 63, 65, 67, 69, 71, 73, 75, 77, 79, 81, 83, 85, 87, 89, 91, 93, 95, 97, 99, 101, 103, 105, 107, 109, 111, 113, 115, 117, 119, 121, 123, 125, 127, 129, 131, 133, 135, 137, and 139; or a polypeptide having 60% or more, for example, 75% or more sequence homology or identity to the amino acid sequence, but is not limited thereto.

Further, as described above, the MFS transporter variant of the present disclosure may comprise a variant in which the amino acid corresponding to position 238 from the N-terminus of SEQ ID NO: 1 (the amino acid at position 231 of SEQ ID NO: 3 and the amino acid at position 216 of SEQ ID NO: 5) is substituted, in connection with SEQ ID NO: 3 and SEQ ID NO: 5, or a variant in which the amino acid corresponding to position 354 from the N-terminus of SEQ ID NO: 1 (the amino acid at position 347 of SEQ ID NO: 3 and the amino acid at position 332 of SEQ ID NO: 5) is substituted, in connection with SEQ ID NO: 3 and SEQ ID NO: 5, and accordingly, it is obvious that the polynucleotide sequence encoding the MFS transporter variant is also comprised in the scope of the present disclosure.

Further, a probe which may be prepared from a known nucleotide sequence, for example, any polynucleotide sequence which hybridizes with a sequence complementary to the entirety or a part of the nucleotide sequence under stringent conditions to encode a protein having the activity of the variant in which an amino acid selected from the group consisting of amino acids corresponding to positions 185, 238, 259, 283, 310, 354, 378, 383, and 403 from the N-terminus of SEQ ID NO: 1 is substituted with another amino acid, may be comprised without limitation.

Still another aspect of the present disclosure provides a vector comprising the polynucleotide encoding the MFS transporter variant of the present disclosure.

Still another aspect of the present disclosure provides a microorganism comprising one or more of the MFS transporter variant, the polynucleotide encoding the variant, and the vector comprising the polynucleotide.

The microorganism comprising one or more of the MFS transporter variant, the polynucleotide encoding the variant, and the vector comprising the polynucleotide may be specifically a microorganism prepared by transforming with the vector comprising the polynucleotide encoding the variant, but is not limited thereto.

The microorganism may be a microorganism expressing the MFS transporter variant.

As used herein, the term "protein allowed to be expressed/to be expressed/expressed" refers to a state in which the target protein is introduced into the microorganism or is modified to be expressed in the microorganism. With respect to the objects of the present disclosure, the "target protein" may be the MFS transporter variant described above.

Specifically, "introduction of the protein" means exhibiting activity of a particular protein that was not originally possessed by a microorganism, or exhibiting enhanced activity, as compared with the endogenous activity of the corresponding protein or the activity before modification. For example, a polynucleotide encoding a particular protein may be introduced into a chromosome in a microorganism or a vector containing a polynucleotide encoding a particular protein may be introduced into a microorganism to exhibit its activity.

The microorganism may be a recombinant microorganism, wherein the recombination may be achieved by genetic modification such as transformation.

In one embodiment of the present disclosure, the microorganism of the present disclosure may have the enhanced MFS transporter activity.

The "enhancement of activity" may mean that activity is improved, as compared with the endogenous activity of a specific protein possessed by a microorganism or activity before modification. The "endogenous activity" may mean the activity of a specific protein originally possessed by a parent strain before the trait is changed, when the trait of the microorganism is changed by genetic variation due to natural or artificial factors.

Specifically, the enhancement of activity of the protein variant in the present disclosure may be achieved by any one or more methods of a method of increasing the intracellular copy number of the gene encoding the protein variant, a method of introducing a variation into the expression regulatory sequence of the gene encoding the protein variant, a method of replacing the expression regulatory sequence of the gene encoding the protein variant by a sequence having strong activity, a method of replacing a gene encoding a natural protein having the MFS transporter activity on the chromosome with the gene encoding the protein variant, and a method of additionally introducing a variation into the gene encoding the variant to enhance the activity of the protein variant, but is not limited thereto.

Next, the modification of the expression regulatory sequence for increasing expression of a polynucleotide may be, but is not particularly limited to, performed by inducing a variation in the sequence via deletion, insertion, non-conservative or conservative substitution, or a combination thereof so as to further enhance the activity of the expression regulatory sequence, or by replacing the nucleotide sequence with a nucleotide sequence with a stronger activity. The expression regulatory sequence may comprise, but is not particularly limited to, a promoter, an operator sequence, a sequence encoding a ribosome-binding domain, and a sequence regulating termination of transcription and translation, etc.

A strong promoter, instead of the original promoter, may be connected to the upstream region of the expression unit of the polynucleotide, but is not limited thereto. Examples of the known strong promoter may comprise cj1 to cj7 promoters (Korean Patent No. 10-0620092), lac promoter, trp promoter, trc promoter, tac promoter, lamda phage PR promoter, PL promoter, tet promoter, gapA promoter, SPL7 promoter, SPL13(sm3) promoter (Korean Patent No. 10-1783170), O2 promoter (Korean Patent No. 10-1632642), tkt promoter, and yccA promoter, etc., and are not limited thereto.

Furthermore, modification of a polynucleotide sequence on chromosome may be, but is not particularly limited to, performed by inducing a variation on the expression regulatory sequence via deletion, insertion, non-conservative or conservative substitution of the nucleotide sequence, or a combination thereof so as to further enhance the activity of the polynucleotide sequence, or by replacing the polynucleotide sequence with a polynucleotide sequence which is improved to have a stronger activity.

Such introduction and enhancement of the protein activity may increase the activity or concentration of the corresponding protein from at least 1%, 10%, 25%, 50%, 75%, 100%, 150%, 200%, 300%, 400% or 500%, to a maximum of 1000% or 2000%, relative to the activity or concentration of a protein of a wild-type or unmodified microbial strain, but is not limited thereto.

In still another embodiment of the present disclosure, the microorganism of the present disclosure may be a microorganism having the purine nucleotide-producing ability.

The microorganism of the present disclosure may be a microorganism having the improved purine nucleotide-producing ability.

As used herein, the term "purine nucleotide" may be any one or more selected from 5'-xanthosine monophosphate (XMP, 5'-xanthylic acid), 5'-guanosine monophosphate (GMP, 5'-guanylic acid), and 5'-inosine monophosphate (IMP, 5'-inosinic acid). Specifically, the purine nucleotide of the present disclosure may be XMP or GMP. XMP refers to a nucleotide resulting from the dehydrogenation of IMP. XMP may be synthesized from IMP by inosine-5'-monophosphate dehydrogenase, but is not limited thereto. GMP has a structure in which a phosphate group forms an ester bond to the ribose moiety of a guanosine molecule, and may be synthesized by adding an ammonia molecule to XMP by 5'-guanylic acid biosynthetic enzyme (GMP synthase), but is not limited to thereto.

The microorganism of the present disclosure may be a microorganism naturally having the purine nucleotide-producing ability or a microorganism in which the purine nucleotide-producing ability is increased or provided by introducing the MFS transporter variant or the polynucleotide encoding the same into a parent strain without the purine nucleotide-producing ability, but is not limited thereto.

With respect to the objects of the present disclosure, the recombinant microorganism of the present disclosure may be a strain of the genus *Corynebacterium,* in which a natural wild-type strain of the genus *Corynebacterium* or a purine nucleotide-producing strain of the genus *Corynebacterium* that comprises the protein having the endogenous MFS transporter activity or the polynucleotide encoding the same is introduced with the MFS transporter variant or the polynucleotide encoding the same to have the increased purine nucleotide-producing ability, as compared to the natural wild-type microorganism or the purine nucleotide-producing strain of the genus *Corynebacterium* that comprises the protein having the endogenous MFS transporter activity or the polynucleotide encoding the same, but is not limited thereto. For example, the natural wild-type strain of the genus *Corynebacterium* or the purine nucleotide-producing strain of the genus *Corynebacterium* that comprises the protein having the endogenous MFS transporter activity or the polynucleotide encoding the same may be a target strain for comparing whether or not the purine nucleotide-producing ability or the MFS transporter activity is increased, as described above, but is not limited thereto.

For example, the recombinant strain having the increased purine nucleotide-producing ability may have an increased purine nucleotide-producing ability of about 0.5% or more, specifically, about 1% or more, about 2% or more, about 3% or more, about 4% or more, about 5% or more, about 6% or more, about 7% or more, about 8% or more, about 9% or more, about 10% or more, about 11% or more, about 12% or more, about 13% or more, about 14% or more, about 15% or more, about 16% or more, about 17% or more, about 18% or more, about 19% or more, about 20% or more, about 21% or more, or about 22% or more (the upper limit is not particularly limited, but may be, for example, about 200% or less, about 150% or less, about 100% or less, about 50% or less, about 40% or less, about 30% or less, about 20% or less, or about 15% or less), as compared to that of the parent strain before modification or unmodified microorganism, but the increased amount is not limited thereto as long as the producing ability has an increased amount of a positive (+) value, as compared to the purine nucleotide-producing ability of the parent strain before modification or the unmodified microorganism. In another example, the microorganism having the increased purine nucleotide-producing ability may have an increased purine nucleotide-producing ability of about 1.005 times or more, about 1.01 times or more, about 1.02 times or more, about 1.03 times or more, about 1.04 times or more, about 1.05 times or more, about 1.06 times or more, about 1.07 times or more, about 1.08 times or more, about 1.09 times or more, about 1.1 times or more, about 1.11 times or more, about 1.12 times or more, about 1.13 times or more, about 1.14 times or more, about 1.15 times or more, about 1.16 times or more, about 1.17 times or more, about 1.18 times or more, about 1.19 times or more, about 1.2 times or more, about 1.21 times or more, or about 1.22 times or more (the upper limit is not particularly limited, but may be, for example, about 10 times or less, about 5 times or less, about 3 times or less, about 2 times or less), as compared to that of the parent strain before modification or the unmodified microorganism, but is not limited thereto.

Such producing ability may be evaluated by measuring the production amount of a target product which is obtained by culturing in a medium. The evaluation may be performed by measuring the production amount of the target product using an appropriate method known in the art. For example, high performance liquid chromatography (HPLC), gas chromatography (GC), gas chromatography-mass spectrometry (GC/MS), liquid chromatography-mass spectrometry (LC/MS), gel permeation chromatography (GPC), or a combination thereof may be used, and the production amount of the target product may be measured using an appropriate method known in the art.

The microorganism of the present disclosure may be any microorganism as long as it expresses the MFS transporter variant by comprising the polynucleotide of the present disclosure or the vector of the present disclosure.

Specifically, the microorganism may be *Corynebacterium stationis, Corynebacterium glutamicum, Corynebacterium crudilactis, Corynebacterium deserti, Corynebacterium efficiens, Corynebacterium callunae, Corynebacterium singulare, Corynebacterium halotolerans, Corynebacterium striatum, Corynebacterium ammoniagenes, Corynebacterium pollutisoli, Corynebacterium imitans, Corynebacterium testudinoris,* or *Corynebacterium flavescens,* specifically, *Corynebacterium stationis* or *Corynebacterium ammoniagenes.* However, the microorganism is not limited thereto, and comprises those taxonomically homologous to the microorganism, regardless of the names of the microorganisms.

In still another embodiment of the present disclosure, the microorganism of the present disclosure may be a microorganism in which the purine nucleotide-producing ability is enhanced by additionally enhancing the activity of some of the proteins involved in the purine nucleotide biosynthesis pathway, or by additionally weakening the activity of some of the proteins involved in the purine nucleotide degradation pathway.

In one embodiment of the above-described embodiments, the recombinant microorganism of the present disclosure may be a microorganism (Korean Patent No. 10-1929158) having enhanced xmpE activity by increasing the copy number of xmpE; replacing the xmpE promoter by a promoter with enhanced activity; replacing the start codon of xmpE; or a combination thereof in order to enhance the purine nucleotide biosynthesis pathway.

In another embodiment of the above-described embodiments, the recombinant microorganism of the present disclosure may be a microorganism (Korean Patent No. 10-2022-0157782) having enhanced phosphate transport system (Pit system) activity, but is not limited thereto.

As used herein, the term "weakening" of the activity of a polypeptide has a concept encompassing all of the reduction of activity or the absence of activity, as compared to the endogenous activity. The weakening may be used interchangeably with the terms such as inactivation, deficiency, down-regulation, decrease, reduction, attenuation, etc.

The weakening may also comprise a case where the activity of the polypeptide itself is reduced or eliminated due to the mutation, etc. of the polynucleotide encoding the polypeptide, as compared to the activity of the polypeptide originally possessed by the microorganism; a case where the activity and/or concentration (expression level) of the overall polypeptides in the cell is low due to the inhibition of the expression of a gene of a polynucleotide encoding the polypeptide or the inhibition of translation into the polypeptide, as compared to the native strain; a case where the expression of the polynucleotide is not made; and/or a case where the polypeptide has no activity even though the polynucleotide is expressed. The "endogenous activity" refers to the activity of a specific polypeptide originally possessed by a parent strain before modification or a wild-type or unmodified microorganism, when the trait is changed by genetic mutation caused by natural or artificial factors. This term may be used interchangeably with the "activity before modification". The "weakening, inactivation, deficiency, decrease, down-regulation, reduction, or attenuation" of the activity of a polypeptide compared to the endogenous activity thereof means that the activity of the polypeptide is lowered compared to the activity of a specific polypeptide originally possessed by a parent strain before transformation or an unmodified microorganism.

Such weakening of the activity of the polypeptide may be performed by any method known in the art, but is not limited thereto, and may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of the polypeptide of the present disclosure may be achieved by:
1) deletion of the entirety or a part of the gene encoding the polypeptide;
2) modification of an expression control region (or expression control sequence) to reduce the expression of a gene encoding the polypeptide;
3) modification of the amino acid sequence constituting the polypeptide to eliminate or weaken the activity of the polypeptide (e.g., deletion/substitution/addition of one or more amino acids in the amino acid sequence);
4) modification of the gene sequence encoding the polypeptide to eliminate or weaken the activity of the polypeptide (e.g., deletion/substitution/addition of one or more nucleotides in the nucleotide sequence of the polypeptide gene to encode the polypeptide which is modified to eliminate or weaken the activity of the polypeptide);
5) modification of a nucleotide sequence encoding an initiation codon, a Shine-Dalgarno sequence, or 5'-UTR region of the gene transcript encoding the polypeptide;
6) introduction of an antisense oligonucleotide (e.g., antisense RNA) complementarily binding to the gene transcript encoding the polypeptide;
7) addition of a sequence complementary to the Shine-Dalgarno sequence of the gene encoding the polypeptide to the upstream of the Shine-Dalgarno sequence in order to form a secondary structure that makes the attachment of ribosomes impossible;
8) addition of a promoter, which is to be reverse-transcribed, to the 3' end of the open reading frame (ORF) of the gene sequence encoding the polypeptide (reverse transcription engineering, RTE);
9) controlling of intracellular localization of the protein (polypeptide); or
10) combination of two or more selected from 1) to 9), but is not particularly limited thereto.

For example,
1) The deletion of a part or the entirety of the gene encoding the polypeptide may be the elimination of the entirety of the polynucleotide encoding an endogenous target protein in the chromosome, replacement with a polynucleotide having deletion of some nucleotides, or replacement with a marker gene.
2) The modification of an expression control region (or expression control sequence) may be the mutation on the expression control region (or expression control sequence) through deletion, insertion, non-conservative or conservative substitution, or a combination thereof, or the replacement with a sequence having weaker activity. The expression control region comprises a promoter, an operator sequence, a sequence for encoding a ribosomal binding site, and a sequence for controlling the termination of transcription and translation, but is not limited thereto.
3) and 4) The modification of the amino acid sequence or the polynucleotide sequence may be the mutation on the sequence through deletion, insertion, non-conservative or conservative substitution, or a combination thereof in the amino acid sequence of the polypeptide or the polynucleotide sequence encoding the polypeptide, or a replacement with an amino acid sequence or polynucleotide sequence that is improved to have weaker activity or an amino acid sequence or polynucleotide sequence improved to have no activity, so as to weaken the activity of the polypeptide, but is not limited thereto. For example, the expression of the gene may be inhibited or attenuated by introducing mutation into the polynucleotide sequence to form a termination codon, but is not limited thereto.
5) The modification of a nucleotide sequence encoding an initiation codon or 5'-UTR region of the gene transcript encoding the polypeptide may be, for example, the substitution with a nucleotide sequence encoding, rather than an endogenous initiation codon, another initiation codon having a lower expression rate of the polypeptide, but is not limited thereto.
6) The introduction of an antisense oligonucleotide (e.g., antisense RNA) complementarily binding to the gene transcript encoding the polypeptide may be referred to, for example, a literature [Weintraub, H. et al., Antisense-RNA as a molecular tool for genetic analysis, Reviews - Trends in Genetics, Vol. 1(1) 1986].
7) The addition of a sequence complementary to the Shine-Dalgarno sequence of the gene encoding the polypeptide to the upstream of the Shine-Dalgarno sequence so as to form a secondary structure that makes the attachment of ribosomes impossible may be making mRNA translation impossible or reducing the rate thereof.

Further, 8) the addition of a promoter, which is to be reverse-transcribed, to the 3' end of the open reading frame (ORF) of the nucleotide sequence encoding the polypeptide (reverse transcription engineering, RTE) may be making an antisense nucleotide complementary to the gene transcript encoding the polypeptide to weaken the activity of the polypeptide.

9) The controlling of intracellular localization of the protein (polypeptide) may mean targeting the protein (polypeptide) to a specific organelle within cells or to a specific intracellular space. For example, targeting to the periplasm or cytoplasm may be achieved through the addition or removal of a leader sequence that functions in targeting the protein (polypeptide), but is not limited thereto.

Such weakening of the activity of the polypeptide may mean that the activity or concentration (expression level) of the corresponding polypeptide is weakened relative to the activity or concentration of the polypeptide expressed in a wild-type or a microbial strain before modification, or that the amount of a product produced from the corresponding polypeptide is decreased, but is not limited thereto.

Still another aspect of the present disclosure provides a method of producing a purine nucleotide, the method comprising the step of culturing, in a medium, the microorganism of the genus *Corynebacterium* comprising any one or more selected from the MFS transporter variant; the polynucleotide encoding the MFS transporter variant; and the vector comprising the polynucleotide.

The method of producing a purine nucleotide of the present disclosure may comprise the step of culturing, in a medium, a microorganism in which the MFS transporter variant or the gene encoding the same is enhanced; or a microorganism which is genetically modified to have the further enhanced MFS transporter variant or the gene encoding the same. The microorganism is as described above.

The MFS transporter variant may have the increased MFS transporter activity, as compared to the wild-type MFS transporter derived from the microorganism of the genus *Corynebacterium.* Alternatively, the MFS transporter variant may not be endogenously expressed in the microorganism of the genus *Corynebacterium.*

The purine nucleotide produced by the culture of the present disclosure may be released into the medium or may remain in cells.

The purine nucleotide may be XMP or GMP, which is as described above.

The method of producing a purine nucleotide of the present disclosure may further comprise the steps of preparing the microorganism of the present disclosure, preparing a medium for culturing the microorganism, or a combination of these steps (regardless of the order, or in any order), for example, before or after the culturing step.

The method of producing a purine nucleotide of the present disclosure may further comprise the step of recovering the purine nucleotide from a medium resulting from the culture (a medium in which culture has been performed) or the cultured microorganism. The recovering step may be further comprised after the culturing step.

The recovering may be the collection of a desired purine nucleotide by using an appropriate method known in the art according to the method of culturing the microorganism of the present disclosure, for example, a batch, continuous, or fed-batch type culture. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, and a desired purine nucleotide may be recovered from the medium or microorganism by using an appropriate method known in the art.

In addition, the method of producing a purine nucleotide of the present disclosure may further comprise a purification step. The purification may be performed by using an appropriate method known in the art. In an exemplary embodiment, when the method of producing a purine nucleotide of the present disclosure comprises both the recovering step and the purification step, the recovering step and the purification step may be performed continuously or discontinuously regardless of the order, or may be performed simultaneously or integrated into one step, but is not limited thereto.

In the method of the present disclosure, the MFS transporter variant, polynucleotide, vector, purine nucleotide and strain, etc. are as described in other aspects.

Still another aspect of the present disclosure provides a composition for producing a purine nucleotide, the composition comprising the microorganism of the genus *Corynebacterium* comprising any one or more selected from the MFS transporter variant, the polynucleotide encoding the MFS transporter variant, and the vector comprising the polynucleotide; a culture medium thereof; or a combination thereof.

The composition of the present disclosure may further comprise any appropriate excipient that is usually used in the composition for producing purine nucleotides, and examples of the excipient may comprise a preserving agent, a wetting agent, a dispersing agent, a suspending agent, a buffer, a stabilizing agent, an isotonic agent, etc., but are not limited thereto.

Still another aspect of the present disclosure provides a method of preparing the purine nucleotide-producing microorganism of the genus *Corynebacterium,* the method comprising the step of introducing any one or more selected from the MFS transporter variant, the polynucleotide encoding the MFS transporter variant, and the vector comprising the polynucleotide into the strain of the genus *Corynebacterium.*

The MFS transporter variant, polynucleotide, vector, purine nucleotide and strain are as described above.

The preparation method may comprise the step of modifying the microorganism to have the increased MFS transporter activity, as compared to the endogenous activity. This comprises introducing the MFS transporter variant into the strain of the genus *Corynebacterium.*

Still another aspect of the present disclosure provides use of the strain of the genus *Corynebacterium,* into which any one or more selected from the MFS transporter variant, the polynucleotide encoding the MFS transporter variant, and the vector comprising the polynucleotide is introduced, in the production of purine nucleotides.

The MFS transporter variant, polynucleotide, vector, purine nucleotide and strain are as described in other aspects.

### [Mode for Carrying Out the Invention]

Hereinafter, the present disclosure will be described in more detail with reference to Examples and Experimental Examples. However, these Examples and Experimental Example are only for illustrating the present disclosure, and the scope of the present disclosure is not intended to be limited by these Examples and Experimental Example.

### Example 1: Construction of random mutation library of major facilitator superfamily (MFS) transporter and Verification of 5'-xanthosine monophosphate (XMP) production ability of MFS transporter mutation-introduced strain

### 1-1. Construction of random mutation library vector

A random mutation library vector was constructed in order to introduce mutations into the endogenous MFS transporter (WP_088859234.1, SEQ ID NO: 1) of *Corynebacterium stationis.*

In detail, a random mutation-induced MFS transporter mutation library was amplified using an error-prone PCR kit (clontech Diversify^{®} PCR Random Mutagenesis Kit), genomic DNA of *Corynebacterium stationis* ATCC6872 strain as a template and a pair of primers of SEQ ID NO: 141 and SEQ ID NO: 142. The amplified product was cloned into pCR vector using a TOPO cloning kit (Invitrogen), which was then transformed into *E*. *coli* DH5α, followed by plating on an LB solid medium containing 25 mg/L of kanamycin. The transformed colonies were taken, plasmids were extracted, which was named a pCR-xmpE random library.

The primer sequences used here are shown in Table 1 below.

**[Table 1]**

| SEQ ID NO. | Sequence name | Sequence (5'->3') |
|---|---|---|
| 141 | xmpE CF | AGCTATGACCATGATTACGCCAAGCTTGGTACCGAGCTCGTTATCATGGGGTGG GTCGCC |
| 142 | xmpE CR | TGAATTGTAATACGACTCACTATAGGGCGAATTGGGCCCTTTATTGAGCCAGAAC CATGG |

### 1-2. Selection of MFS transporter mutation-introduced strain and Verification of XMP production ability

The pCR-xmpE random library produced in Example 1-1 was transformed into an XMP-producing *Corynebacterium ammoniagenes* KCCM10530 strain (Korean Patent No. 10-0542568) by electroporation (Appl. Microbiol.Biotechnol. (1999) 52: 541-545) and plated on a nutrient medium containing 25 mg/L of kanamycin. 5,000 colonies in which mutations were introduced into the MFS transporter were obtained therefrom, and each colony was named from KCCM10530_pCR-xmpE(mt)1 to KCCM10530_pCR-xmpE(mt)5000.

The composition of the nutrient medium is as follows.

### <XMP nutrient medium>

1% glucose, 1% peptone, 1% beef extract, 0.25% sodium chloride, 1% yeast extract, 100 mg/L adenine, 100 mg/L guanine, 2% agar, pH 7.2 (based on 1 liter of distilled water)

The obtained 15,000 colonies were each autoclaved and then inoculated into a 96 deep well plate containing 100 µl of a seed medium containing 25 mg/L of kanamycin and incubated in a microplate shaker (TAITEC) under shaking at 1200 rpm at 30°C for 24 hours, which was used as a seed culture. 320 µl of an autoclaved production medium below (240 µl of main medium + 80 µl of separate sterile medium) was dispensed into a 96 deep well plate, then 15 µl of the seed culture was inoculated and incubated under shaking for 72 hours under the same conditions as above.

The compositions of the seed medium, main medium and separate sterile medium are as follows.

### <XMP flask seed medium>

30g/L glucose, 15 g/L peptone, 15 g/L yeast extract, 2.5 g/L sodium chloride, 3 g/L urea, 150 mg/L adenine, 150 mg/L guanine, pH 7.0 (based on 1 liter of distilled water)

### <XMP flask production medium (main medium)>

50 g/L glucose, 10 g/L magnesium sulfate, 3 g/L yeast extract, 100 mg/L calcium chloride, 20 mg/L iron sulfate, 10 mg/L manganese sulfate, 10 mg/L zinc sulfate, 0.8 mg/L copper sulfate, 20 mg/L histidine, 15 mg/L cystine, 15 mg/L beta-alanine, 100 ug/L biotin, 5 mg/L thiamine, 50 mg/L adenine, 25 mg/L guanine, 15 mg/L niacin, pH 7.0 (based on 1 liter of distilled water)

### <XMP flask production medium (separate sterile medium)>

18 g/L potassium phosphate monobasic, 42 g/L potassium phosphate dibasic, 7 g/L urea, 5 g/L ammonium sulfate (based on 1 liter of distilled water)

In order to analyze the production amount of XMP in the culture medium, after completion of the culture, 640 µL of sterile water was dispensed into the culture medium and centrifuged at 4000 rpm for 15 minutes, and then 3 µL of the supernatant was transferred to a 96-well UV plate into which 297 µL of distilled water was dispensed. Stirring was performed for 30 seconds using a microplate reader, then absorbance was measured using a spectrophotometer at 25°C and a wavelength of 260 nm, and 12 types of mutant strains with increased absorbance (KCCM10530_pCR-xmpE(mt)_559, KCCM10530_pCR-xmpE(mt)_618, KCCM10530_pCR-xmpE(mt)_1218, KCCM10530_pCR-xmpE(mt)_1512, KCCM10530_pCR-xmpE(mt)_2372, KCCM10530_pCR-xmpE(mt)_2826, KCCM10530_pCR-xmpE(mt)_3194, KCCM10530_pCR-xmpE(mt)_3451, KCCM10530_pCR-xmpE(mt)_4028, KCCM10530_pCR-xmpE(mt)_4317, KCCM10530_pCR-xmpE(mt)_4737, KCCM10530_pCR-xmpE(mt)_4888), as compared to the control strain, were selected, excluding mutant strains with the similar or reduced level of absorbance, as compared to the control strain (KCCM10530).

### 1-3. Verification of XMP production ability of selected MFS transporter mutation-introduced strain

Fermentation titer was evaluated to verify the effectiveness of XMP production from the selected strains. KCCM10530 as a control strain and 12 type of mutant strains selected above were inoculated into a 14 mL tube containing 2.5 mL of the seed medium, respectively, and cultured under shaking at 170 rpm at 30°C for 24 hours. 0.7 mL of seed culture was inoculated into a 250 mL corner-baffled flask containing 32 mL of the production medium (24 mL of main medium + 8 mL of separate sterile medium) and cultured under shaking at 170 rpm at 30°C for 75 hours. After completion of the culture, the production amount of XMP was measured using HPLC, and the results are shown in Table 2 below.

**[Table 2]**

| Name of strain | | Mutation | XMP concentration (g/L) |
|---|---|---|---|
| Control | KCCM10530 | WT | 10.9 |
| 1 | KCCM10530_pCR-xmpE(mt)_559 | F354S | 11.9 |
| 2 | KCCM10530_pCR-xmpE(mt)_618 | L185Q | 11.4 |
| 3 | KCCM10530_pCR-xmpE(mt)_1218 | V378A | 11.7 |
| 4 | KCCM10530_pCR-xmpE(mt)_1512 | 1383L | 11.6 |
| 5 | KCCM10530_pCR-xmpE(mt)_2372 | F354Y | 12.2 |
| 6 | KCCM10530_pCR-xmpE(mt)_2826 | 1238T | 12.0 |
| 7 | KCCM10530_pCR-xmpE(mt)_3194 | 1259T | 14.1 |

| | | | |
|---|---|---|---|
| 8 | KCCM10530_pCR-xmpE(mt)_3451 | F354N | 13.2 |
| 9 | KCCM10530_pCR-xmpE(mt)_4028 | V403A | 11.6 |
| 10 | KCCM10530_pCR-xmpE(mt)_4317 | S283G | 11.3 |
| 11 | KCCM10530_pCR-xmpE(mt)_4737 | 1259S | 11.4 |
| 12 | KCCM10530_pCR-xmpE(mt)_4888 | V310A | 11.5 |

As shown in Table 2, 12 types of mutant strains in which mutations were introduced into the MFS transporter and absorbance was increased were confirmed to have the increased XMP production ability, as compared to the control strain KCCM10530.

To verify the mutant sequence introduced on the MFS transporter of the 12 strains with the increased XMP production ability, PCR and sequencing analysis were performed on each strain using a pair of primer of SEQ ID NO: 143 and SEQ ID NO: 144.

The primer sequences used here are shown in Table 3 below.

**[Table 3]**

| SEQ ID NO. | Sequence name | Sequence (5'->3') |
|---|---|---|
| 143 | xmpE seq F | GGAATCCGCTGTCATGATCT |
| 144 | xmpE seq R | TCGCAGTTCACGCACTAAAG |

As a result of comparing the polynucleotide sequences of the gene fragments encoding the MFS transporter variants derived from the obtained 12 strains with the nucleotide sequence encoding the MFS transporter derived from the control strain KCCM10530, it was confirmed that KCCM10530_pCR-xmpE(mt)_559 comprised a mutation of substituting phenylalanine (F), which is an amino acid at position 354 from the N-terminus of the MFS transporter, with serine (S), KCCM10530_pCR-xmpE(mt)_618 comprised a mutation of substituting leucine (L), which is an amino acid at position 185 from the N-terminus of the MFS transporter, with glutamine (Q), KCCM10530_pCR-xmpE(mt)_1218 comprised a mutation of substituting valine (V), which is an amino acid at position 378 from the N-terminus of the MFS transporter, with alanine (A), KCCM10530_pCR-xmpE(mt)_1512 comprised a mutation of substituting isoleucine (I), which is an amino acid at position 383 from the N-terminus of the MFS transporter, with leucine (L), KCCM10530_pCR-xmpE(mt)_2372 comprised a mutation of substituting phenylalanine (F), which is an amino acid at position 354 from the N-terminus of the MFS transporter, with tyrosine (Y), KCCM10530_pCR-xmpE(mt)_2826 comprised a mutation of substituting isoleucine (I), which is an amino acid at position 238 from the N-terminus of the MFS transporter, with threonine (T), KCCM10530_pCR-xmpE(mt)_3194 comprised a mutation of substituting isoleucine (I), which is an amino acid at position 259 from the N-terminus of the MFS transporter, with threonine (T), KCCM10530_pCR-xmpE(mt)_3451 comprised a mutation of substituting phenylalanine (F), which is an amino acid at position 354 from the N-terminus of the MFS transporter, with asparagine (N), KCCM10530_pCR-xmpE(mt)_4028 comprised a mutation of substituting valine (V), which is an amino acid at position 403 from the N-terminus of the MFS transporter, with alanine (A), KCCM10530_pCR-xmpE(mt)_4317 comprised a mutation of substituting serine (S), which is an amino acid at position 283 from the N-terminus of the MFS transporter, with glycine (G), KCCM10530_pCR-xmpE(mt)_4737 comprised a mutation of substituting isoleucine (I), which is an amino acid at position 259 from the N-terminus of the MFS transporter, with serine (S), and KCCM10530_pCR-xmpE(mt)_4888 comprised a mutation of substituting valine (V), which is an amino acid at position 310 from the N-terminus of the MFS transporter, with alanine (A).

### Example 2: Vector for introducing MFS transporter mutation and Verification of XMP production ability of MFS transporter mutation-introduced Corynebacterium stationis

### 2-1. Construction of vector for introducing MFS transporter mutation

In order to introduce the MFS transporter mutations, which were identified in the 12 strains with the increased XMP production ability in Example 1-2, into the endogenous MFS transporter of *Corynebacterium stationis,* vectors were constructed for the introduction of 12 MFS transporter.

In detail, PCR was performed using each chromosome of KCCM10530_pCR-xmpE(mt)_559, KCCM10530_pCR-xmpE(mt)_618, KCCM10530_pCR-xmpE(mt)_1218, KCCM10530_pCR-xmpE(mt)_1512, KCCM10530_pCR-xmpE(mt)_2372, KCCM10530_pCR-xmpE(mt)_2826, KCCM10530_pCR-xmpE(mt)_3194, KCCM10530_pCR-xmpE(mt)_3451, KCCM10530_pCR-xmpE(mt)_4028, KCCM10530_pCR-xmpE(mt)_4317, KCCM10530_pCR-xmpE(mt)_4737, KCCM10530_pCR-xmpE(mt)_4888 as a template and a pair of primers of SEQ ID NO: 143 and SEQ ID NO: 144.

At this time, Solg^{™} Pfu-X DNA polymerase was used as polymerase, and PCR amplification was performed under conditions of denaturation at 95°C for 5 minutes, followed by 30 cycles of denaturation at 95°C for 30 seconds, annealing at 58°C for 30 seconds, and polymerization at 72°C for 60 seconds, and polymerization at 72°C for 5 minutes.

Each PCR product amplified above, and a vector pDCM2 (Korean Patent Publication No. 10-2020-0136813) for insertion and replacement of the gene into the chromosome of *Corynebacterium,* digested with Xbal restriction enzyme, were cloned using Gibson assembly (DG Gibson et al., NATURE METHODS, VOL.6 NO.5, MAY 2009, NEBuilder HiFi DNA Assembly Master Mix) to obtain each recombinant vector. Cloning was performed by mixing a Gibson assembly reagent and each gene fragment in the calculated molar quantity and storing the mixture at 50°C for 1 hour. 12 types of recombinant vectors thus constructed were named pDCM2-xmpE(F354S), pDCM2-xmpE(L185Q), pDCM2-xmpE(V378A), pDCM2-xmpE(I383L), pDCM2-xmpE(F354Y), pDCM2-xmpE(I238T), pDCM2-xmpE(I259T), pDCM2-xmpE(F354N), pDCM2-xmpE(V403A), pDCM2-xmpE(S283G), pDCM2-xmpE(I259S), and pDCM2-xmpE(V310A) vectors, respectively.

### 2-2. Preparation of MFS transporter mutation-introduced Corynebacterium stationis

12 recombinant vectors constructed in Example 2-1 were respectively transformed into an XMP-producing *Corynebacterium stationis* KCCM12248P strain (Korean Patent No. 10-1929158) by electroporation to obtain each strain in which each vector was inserted into the chromosome by recombination of homologous sequence. Each strain was selected on a medium containing 25 mg/L of kanamycin. Each selected primary strain was again subjected to secondary cross-over, and strains were selected, in which the mutation was introduced into the gene encoding the MFS transporter. The corresponding genetic manipulation was identified through PCR and sequencing analysis using a pair of primers of SEQ ID NO: 143 and SEQ ID NO: 144.

12 strains obtained above were named KCCM12248P::xmpE(F354S), KCCM12248P::xmpE(L185Q), KCCM12248P::xmpE(V378A), KCCM12248P::xmpE(1383L), KCCM12248P::xmpE(F354Y), KCCM12248P::xmpE(1238T), KCCM12248P::xmpE(1259T), KCCM12248P::xmpE(F354N), KCCM12248P::xmpE(V403A), KCCM12248P::xmpE(S283G), KCCM12248P::xmpE(I259S), and KCCM12248P::xmpE(V310A), respectively.

### 2-3. Verification of XMP production ability of MFS transporter mutation-introduced Corynebacterium stationis

XMP production abilities of 12 strains prepared in Example 2-2, KCCM12248P::xmpE(F354S), KCCM12248P::xmpE(L185Q), KCCM12248P::xmpE(V378A), KCCM12248P::xmpE(I383L), KCCM12248P::xmpE(F354Y), KCCM12248P::xmpE(I238T), KCCM12248P::xmpE(I259T), KCCM12248P::xmpE(F354N), KCCM12248P::xmpE(V403A), KCCM12248P::xmpE(S283G), KCCM12248P::xmpE(I259S), and KCCM12248P::xmpE(V310A), and KCCM10530, KCCM12248P were examined by the fermentation titer evaluation method of Example 1-3. After completion of the culture, the production amounts of XMP were measured using HPLC, and the culture results are shown in Table 4 below.

**[Table 4]**

| | Name of strain | XMP concentration (g/L) | XMP yield (%) |
|---|---|---|---|
| Control | KCCM10530 | 10.9 | 21.8 |
| Control | KCCM12248P | 13.5 | 27.0 |
| 1 | KCCM12248P::xmpE(F354S) | 15.9 | 31.8 |
| 2 | KCCM12248P::xmpE(L185Q) | 14.3 | 28.6 |
| 3 | KCCM12248P::xmpE(V378A) | 14.7 | 29.4 |
| 4 | KCCM12248P::xmpE(I383L) | 14.5 | 29.0 |
| 5 | KCCM12248P::xmpE(F354Y) | 15.6 | 31.2 |
| 6 | KCCM12248P::xmpE(I238T) | 15.3 | 30.6 |
| 7 | KCCM12248P::xmpE(I259T) | 14.6 | 29.2 |
| 8 | KCCM12248P::xmpE(F354N) | 16.5 | 33.0 |
| 9 | KCCM12248P::xmpE(V403A) | 14.2 | 28.4 |
| 10 | KCCM12248P::xmpE(S283G) | 15.4 | 30.8 |
| 11 | KCCM12248P::xmpE(1259S) | 14.9 | 29.8 |
| 12 | KCCM12248P::xmpE(V310A) | 14.5 | 29.0 |

As shown in Table 4, the *Corynebacterium stationis* strains, into which the MFS transporter mutation was introduced, had the significantly increased XMP production ability, as compared to the control strain, KCCM10530. In particular, it was confirmed that the XMP production ability increased by 5% to 22% or more, as compared to that of the parent strain, KCCM12248P.

### Example 3: Vector for introducing mutation at F354 position of MFS transporter and Verification of XMP production ability of MFS transporter mutation-introduced Corynebacterium stationis

### 3-1. Construction of vector for introducing MFS transporter mutation

In Example 2-3, it was confirmed that the XMP production ability may be increased when the amino acid at position 354 from the N-terminus of the MFS transporter is mutated. In order to construct a vector for substituting the amino acid at position 354 with another amino acid, site-directed mutagenesis was performed using the pDCM2-xmpE(F354S) vector constructed in Example 2-1 as a template.

In detail, site-directed PCR was performed using a pair of primers of SEQ ID NO: 145 and SEQ ID NO: 146 for introducing F354C mutation into the gene encoding the MFS transporter, a pair of primers of SEQ ID NO: 147 and SEQ ID NO: 148 for introducing F354R mutation, a pair of primers of SEQ ID NO: 149 and SEQ ID NO: 150 for introducing F354I mutation, a pair of primers of SEQ ID NO: 151 and SEQ ID NO: 152 for introducing F354V mutation, a pair of primers of SEQ ID NO: 153 and SEQ ID NO: 154 for introducing F354D mutation, a pair of primers of SEQ ID NO: 155 and SEQ ID NO: 156 for introducing F354G mutation, a pair of primers of SEQ ID NO: 157 and SEQ ID NO: 158 for introducing F354P mutation, a pair of primers of SEQ ID NO: 159 and SEQ ID NO: 160 for introducing F354A mutation, a pair of primers of SEQ ID NO: 161 and SEQ ID NO: 162 for introducing F354E mutation, a pair of primers of SEQ ID NO: 163 and SEQ ID NO: 164 for introducing F354M mutation, a pair of primers of SEQ ID NO: 165 and SEQ ID NO: 166 for introducing F354W mutation, a pair of primers of SEQ ID NO: 167 and SEQ ID NO: 168 for introducing F354T mutation, a pair of primers of SEQ ID NO: 169 and SEQ ID NO: 170 for introducing F354Q mutation, a pair of primers of SEQ ID NO: 171 and SEQ ID NO: 172 for introducing F354K mutation, a pair of primers of SEQ ID NO: 173 and SEQ ID NO: 174 for introducing F354L mutation, a pair of primers of SEQ ID NO: 175 and SEQ ID NO: 176 for introducing F354H mutation, respectively.

The primer sequences used here are shown in Table 5 below.

**[Table 5]**

| SEQ ID NO. | Sequence name | Sequence (5'->3') |
|---|---|---|
| 145 | pDCM2-xmpE(F354C) | CGTTGACCTCGGCTGCGGCTTCGGCCCAGT |
| 146 | pDCM2-xmpE(F354C) | ACTGGGCCGAAGCCGCAGCCGAGGTCAACG |
| 147 | pDCM2-xmpE(F354R) | CGTTGACCTCGGCAGAGGCTTCGGCCCAGT |
| 148 | pDCM2-xmpE(F354R) | ACTGGGCCGAAGCCTCTGCCGAGGTCAACG |
| 149 | pDCM2-xmpE(F354I) | CGTTGACCTCGGCATCGGCTTCGGCCCAGT |
| 150 | pDCM2-xmpE(F354I) | ACTGGGCCGAAGCCGATGCCGAGGTCAACG |
| 151 | pDCM2-xmpE(F354V) | CGTTGACCTCGGCGTGGGCTTCGGCCCAGT |
| 152 | pDCM2-xmpE(F354V) | ACTGGGCCGAAGCCCACGCCGAGGTCAACG |
| 153 | pDCM2-xmpE(F354D) | CGTTGACCTCGGCGATGGCTTCGGCCCAGT |
| 154 | pDCM2-xmpE(F354D) | ACTGGGCCGAAGCCATCGCCGAGGTCAACG |
| 155 | pDCM2-xmpE(F354G) | CGTTGACCTCGGCGGCGGCTTCGGCCCAGT |
| 156 | pDCM2-xmpE(F354G) | ACTGGGCCGAAGCCGCCGCCGAGGTCAACG |
| 157 | pDCM2-xmpE(F354P) | CGTTGACCTCGGCCCAGGCTTCGGCCCAGT |
| 158 | pDCM2-xmpE(F354P) | ACTGGGCCGAAGCCTGGGCCGAGGTCAACG |
| 159 | pDCM2-xmpE(F354A) | CGTTGACCTCGGCGCAGGCTTCGGCCCAGT |
| 160 | pDCM2-xmpE(F354A) | ACTGGGCCGAAGCCTGCGCCGAGGTCAACG |
| 161 | pDCM2-xmpE(F354E) | CGTTGACCTCGGCGAAGGCTTCGGCCCAGT |
| 162 | pDCM2-xmpE(F354E) | ACTGGGCCGAAGCCTTCGCCGAGGTCAACG |
| 163 | pDCM2-xmpE(F354M) | CGTTGACCTCGGCATGGGCTTCGGCCCAGT |
| 164 | pDCM2-xmpE(F354M) | ACTGGGCCGAAGCCCATGCCGAGGTCAACG |
| 165 | pDCM2-xmpE(F354W) | CGTTGACCTCGGCTGGGGCTTCGGCCCAGT |
| 166 | pDCM2-xmpE(F354W) | ACTGGGCCGAAGCCCCAGCCGAGGTCAACG |
| 167 | pDCM2-xmpE(F354T) | CGTTGACCTCGGCACCGGCTTCGGCCCAGT |
| 168 | pDCM2-xmpE(F354T) | ACTGGGCCGAAGCCGGTGCCGAGGTCAACG |
| 169 | pDCM2-xmpE(F354Q) | CGTTGACCTCGGCCAGGGCTTCGGCCCAGT |
| 170 | pDCM2-xmpE(F354Q) | ACTGGGCCGAAGCCCTGGCCGAGGTCAACG |
| 171 | pDCM2-xmpE(F354K) | CGTTGACCTCGGCAAGGGCTTCGGCCCAGT |
| 172 | pDCM2-xmpE(F354K) | ACTGGGCCGAAGCCCTTGCCGAGGTCAACG |
| 173 | pDCM2-xmpE(F354L) | CGTTGACCTCGGCCTGGGCTTCGGCCCAGT |
| 174 | pDCM2-xmpE(F354L) | ACTGGGCCGAAGCCCAGGCCGAGGTCAACG |
| 175 | pDCM2-xmpE(F354H) | CGTTGACCTCGGCCACGGCTTCGGCCCAGT |
| 176 | pDCM2-xmpE(F354H) | ACTGGGCCGAAGCCGTGGCCGAGGTCAACG |

At this time, Solg^{™} Pfu-X DNA polymerase was used as polymerase, and PCR amplification was performed under conditions of denaturation at 95°C for 5 minutes, followed by 24 cycles of denaturation at 95°C for 30 seconds, annealing at 58°C for 30 seconds, and polymerization at 72°C for 10 minutes, and polymerization at 72°C for 5 minutes.

The PCR product amplified above was treated with Dpnl restriction enzyme to remove pDCM2-xmpE(F354S) which was used as the template, and then transformed into *E*. *coli* DH5a to obtain a vector in which the amino acid at position 354 of the MFS transporter was modified with the desired amino acid. 16 vectors obtained above were named pDCM2-xmpE(F354C), pDCM2-xmpE(F354R), pDCM2-xmpE(F354I), pDCM2-xmpE(F35V), pDCM2-xmpE(F354D), pDCM2-xmpE(F354G), pDCM2-xmpE(F354P), pDCM2-xmpE(F354A), pDCM2-xmpE(F354E), pDCM2-xmpE(F354M), pDCM2-xmpE(F354W), pDCM2-xmpE(F354T), pDCM2-xmpE(F354Q), pDCM2-xmpE(F354K), pDCM2-xmpE(F354L), and pDCM2-xmpE(F354H), respectively.

### 3-2. Preparation of MFS transporter mutation-introduced Corynebacterium stationis

16 vectors constructed in Example 3-1 were respectively transformed into *Corynebacterium stationis* KCCM12248P strain by electroporation to obtain each strain in which each vector was inserted into the chromosome by recombination of homologous sequence. Each strain was selected on a medium containing 25 mg/L of kanamycin. Each selected primary strain was again subjected to secondary cross-over, and strains were selected, in which the amino acid at position 354 of the MFS transporter was modified with the desired amino acid. The corresponding genetic manipulation was identified through PCR using a pair of primers of SEQ ID NO: 141 and SEQ ID NO: 142 and sequencing analysis using a pair of primers of SEQ ID NO: 143 and SEQ ID NO: 144.

16 strains obtained above were named KCCM12248P::xmpE(F354C), KCCM12248P::xmpE(F354R), KCCM12248P::xmpE(F354I), KCCM12248P::xmpE(F354V), KCCM12248P::xmpE(F354D), KCCM12248P::xmpE(F354G), KCCM12248P::xmpE(F354P), KCCM12248P::xmpE(F354A), KCCM12248P::xmpE(F354E), KCCM12248P::xmpE(F354M), KCCM12248P::xmpE(F354W), KCCM12248P::xmpE(F354T), KCCM12248P::xmpE(F354Q), KCCM12248P::xmpE(F354K), KCCM12248P::xmpE(F354L), and KCCM12248P::xmpE(F354H), respectively.

### 3-3. Verification of XMP production ability of MFS transporter mutation-introduced Corynebacterium stationis

16 strains prepared in Example 3-2, KCCM12248P::xmpE(F354S) strain, KCCM12248P::xmpE(F354Y) strain, and KCCM12248P::xmpE(F354N) strain prepared in Example 2-2, and control KCCM12248P strain were cultured by the fermentation titer evaluation method of Example 1-3. After completion of the culture, the production amounts of XMP were measured using HPLC, and the results are shown in Table 6 below.

**[Table 6]**

| | Name of strain | XMP concentration (g/L) |
|---|---|---|
| 1 | KCCM12248P | 13.5 |
| 2 | **KCCM12248P::xmpE(F354S)** | **16.1** |
| 3 | **KCCM12248P::xmpE(F354Y)** | **15.7** |
| 4 | **KCCM12248P::xmpE(F354N)** | **16.6** |
| 5 | **KCCM12248P::xmpE(F354L)** | **14.4** |

As shown in Table 6, it was confirmed that the *Corynebacterium stationis* strains, each in which the amino acid at position 354 from the N-terminus of the MFS transporter was substituted with serine, tyrosine, asparagine, or leucine, had 6% to 22% or more increase in the XMP production ability, as compared to the parent strain, KCCM12248P.

### Example 4: Vector for introducing mutation at 1259 position of MFS transporter and Verification of XMP production ability of MFS transporter mutation-introduced Corynebacterium stationis

### 4-1. Construction of vector for introducing MFS transporter mutation

In Example 2-3, it was confirmed that the XMP production ability may be increased when the amino acid at position 259 from the N-terminus of the MFS transporter is mutated. In order to construct a vector for substituting the amino acid at position 259 with another amino acid, site-directed mutagenesis was performed using the pDCM2-xmpE(I259T) vector constructed in Example 2-1 as a template.

In detail, site-directed PCR was performed using a pair of primers of SEQ ID NO: 177 and SEQ ID NO: 178 for introducing I259F mutation into the gene encoding the MFS transporter, a pair of primers of SEQ ID NO: 179 and SEQ ID NO: 180 for introducing I259Y mutation, a pair of primers of SEQ ID NO: 181 and SEQ ID NO: 182 for introducing I259C mutation, a pair of primers of SEQ ID NO: 183 and SEQ ID NO: 184 for introducing I259L mutation, a pair of primers of SEQ ID NO: 185 and SEQ ID NO: 186 for introducing I259H mutation, a pair of primers of SEQ ID NO: 187 and SEQ ID NO: 188 for introducing I259V mutation, a pair of primers of SEQ ID NO: 189 and SEQ ID NO: 190 for introducing I259D mutation, a pair of primers of SEQ ID NO: 191 and SEQ ID NO: 192 for introducing I259G mutation, a pair of primers of SEQ ID NO: 193 and SEQ ID NO: 194 for introducing I259P mutation, a pair of primers of SEQ ID NO: 195 and SEQ ID NO: 196 for introducing I259A mutation, a pair of primers of SEQ ID NO: 197 and SEQ ID NO: 198 for introducing I259E mutation, a pair of primers of SEQ ID NO: 199 and SEQ ID NO: 200 for introducing I259M mutation, a pair of primers of SEQ ID NO: 201 and SEQ ID NO: 202 for introducing I259W mutation, a pair of primers of SEQ ID NO: 203 and SEQ ID NO: 204 for introducing I259Q mutation, a pair of primers of SEQ ID NO: 205 and SEQ ID NO: 206 for introducing I259K mutation, a pair of primers of SEQ ID NO: 207 and SEQ ID NO: 208 for introducing I259R mutation, and a pair of primers of SEQ ID NO: 209 and SEQ ID NO: 210 for introducing I259N mutation, respectively.

The primer sequences used here are shown in Table 7 below.

**[Table 7]**

| SEQ ID NO. | Sequence name | Sequence (5'->3') |
|---|---|---|
| 177 | pDCM2-xmpE(I259F) | CGCAGGCCTATTCTTTTTCGCTTACGCCATCTC |
| 178 | pDCM2-xmpE(I259F) | GAGATGGCGTAAGCGAAAAAGAATAGGCCTGCG |
| 179 | pDCM2-xmpE(I259Y) | CGCAGGCCTATTCTTTTACGCTTACGCCATCTC |
| 180 | pDCM2-xmpE(I259Y) | GAGATGGCGTAAGCGTAAAAGAATAGGCCTGCG |
| 181 | pDCM2-xmpE(I259C) | CGCAGGCCTATTCTTTTGCGCTTACGCCATCTC |
| 182 | pDCM2-xmpE(I259C) | GAGATGGCGTAAGCGCAAAAGAATAGGCCTGCG |
| 183 | pDCM2-xmpE(I259L) | CGCAGGCCTATTCTTTCTGGCTTACGCCATCTC |
| 184 | pDCM2-xmpE(I259L) | GAGATGGCGTAAGCCAGAAAGAATAGGCCTGCG |
| 185 | pDCM2-xmpE(I259H) | CGCAGGCCTATTCTTTCACGCTTACGCCATCTC |
| 186 | pDCM2-xmpE(I259H) | GAGATGGCGTAAGCGTGAAAGAATAGGCCTGCG |
| 187 | pDCM2-xmpE(I259V) | CGCAGGCCTATTCTTTGTGGCTTACGCCATCTC |
| 188 | pDCM2-xmpE(I259V) | GAGATGGCGTAAGCCACAAAGAATAGGCCTGCG |
| 189 | pDCM2-xmpE(I259D) | CGCAGGCCTATTCTTTGATGCTTACGCCATCTC |
| 190 | pDCM2-xmpE(I259D) | GAGATGGCGTAAGCATCAAAGAATAGGCCTGCG |
| 191 | pDCM2-xmpE(I259G) | CGCAGGCCTATTCTTTGGCGCTTACGCCATCTC |
| 192 | pDCM2-xmpE(I259G) | GAGATGGCGTAAGCGCCAAAGAATAGGCCTGCG |
| 193 | pDCM2-xmpE(I259P) | CGCAGGCCTATTCTTTCCAGCTTACGCCATCTC |
| 194 | pDCM2-xmpE(I259P) | GAGATGGCGTAAGCTGGAAAGAATAGGCCTGCG |
| 195 | pDCM2-xmpE(I259A) | CGCAGGCCTATTCTTTGCAGCTTACGCCATCTC |
| 196 | pDCM2-xmpE(I259A) | GAGATGGCGTAAGCTGCAAAGAATAGGCCTGCG |
| 197 | pDCM2-xmpE(I259E) | CGCAGGCCTATTCTTTGAAGCTTACGCCATCTC |
| 198 | pDCM2-xmpE(I259E) | GAGATGGCGTAAGCTTCAAAGAATAGGCCTGCG |
| 199 | pDCM2-xmpE(I259M) | CGCAGGCCTATTCTTTATGGCTTACGCCATCTC |
| 200 | pDCM2-xmpE(I259M) | GAGATGGCGTAAGCCATAAAGAATAGGCCTGCG |
| 201 | pDCM2-xmpE(I259W) | CGCAGGCCTATTCTTTTGGGCTTACGCCATCTC |
| 202 | pDCM2-xmpE(I259W) | GAGATGGCGTAAGCCCAAAAGAATAGGCCTGCG |
| 203 | pDCM2-xmpE(I259Q) | CGCAGGCCTATTCTTTCAGGCTTACGCCATCTC |
| 204 | pDCM2-xmpE(I259Q) | GAGATGGCGTAAGCCTGAAAGAATAGGCCTGCG |
| 205 | pDCM2-xmpE(I259K) | CGCAGGCCTATTCTTTAAGGCTTACGCCATCTC |
| 206 | pDCM2-xmpE(I259K) | GAGATGGCGTAAGCCTTAAAGAATAGGCCTGCG |
| 207 | pDCM2-xmpE(I259R) | CGCAGGCCTATTCTTTAGAGCTTACGCCATCTC |
| 208 | pDCM2-xmpE(I259R) | GAGATGGCGTAAGCTCTAAAGAATAGGCCTGCG |
| 209 | pDCM2-xmpE(I259N) | CGCAGGCCTATTCTTTAACGCTTACGCCATCTC |
| 210 | pDCM2-xmpE(I259N) | GAGATGGCGTAAGCGTTAAAGAATAGGCCTGCG |

At this time, Solg^{™} Pfu-X DNA polymerase was used as polymerase, and PCR amplification was performed under conditions of denaturation at 95°C for 5 minutes, followed by 24 cycles of denaturation at 95°C for 30 seconds, annealing at 58°C for 30 seconds, and polymerization at 72°C for 10 minutes, and polymerization at 72°C for 5 minutes.

The PCR product amplified above was treated with Dpnl restriction enzyme to remove pDCM2-xmpE(I259T) vector which was used as the template, and then transformed into E. *coli* DH5a to obtain a vector in which the amino acid at position 259 of the MFS transporter was modified with the desired amino acid. 17 vectors obtained above were named pDCM2-xmpE(I259F), pDCM2-xmpE(I259Y), pDCM2-xmpE(I259C), pDCM2-xmpE(I259L), pDCM2-xmpE(I259H), pDCM2-xmpE(I259V), pDCM2-xmpE(I259D), pDCM2-xmpE(I259G), pDCM2-xmpE(I259P), pDCM2-xmpE(I259A), pDCM2-xmpE(I259E), pDCM2-xmpE(I259M), pDCM2-xmpE(I259W), pDCM2-xmpE(I259Q), pDCM2-xmpE(I259K), pDCM2-xmpE(I259R), and pDCM2-xmpE(I259N), respectively.

### 4-2. Preparation of MFS transporter mutation-introduced Corynebacterium stationis

17 vectors constructed in Example 4-1 were respectively transformed into *Corynebacterium stationis* KCCM12248P strain by electroporation to obtain each strain in which each vector was inserted into the chromosome by recombination of homologous sequence. Each strain was selected on a medium containing 25 mg/L of kanamycin. Each selected primary strain was again subjected to secondary cross-over, and strains were selected, in which the amino acid at position 259 of the MFS transporter was modified with the desired amino acid. The corresponding genetic manipulation was identified through PCR using a pair of primers of SEQ ID NO: 141 and SEQ ID NO: 142 and sequencing analysis using a pair of primers of SEQ ID NO: 143 and SEQ ID NO: 144.

17 strains obtained above were named KCCM12248P::xmpE(I259F), KCCM12248P::xmpE(I259Y), KCCM12248P::xmpE(I259C), KCCM12248P::xmpE(I259L), KCCM12248P::xmpE(I259H), KCCM12248P::xmpE(I259V), KCCM12248P::xmpE(I259D), KCCM12248P::xmpE(I259G), KCCM12248P::xmpE(I259P), KCCM12248P::xmpE(I259A), KCCM12248P::xmpE(I259E), KCCM12248P::xmpE(I259M), KCCM12248P::xmpE(I259W), KCCM12248P::xmpE(I259Q), KCCM12248P::xmpE(I259K), KCCM12248P::xmpE(I259R), and KCCM12248P::xmpE(I259N), respectively.

### 4-3. Verification of XMP production ability of MFS transporter mutation-introduced Corynebacterium stationis

The strains prepared in Example 4-2, KCCM12248P::xmpE(1259S) strain and KCCM12248P::xmpE(I259T) strain prepared in Example 2-2, and control KCCM12248P strain were cultured by the fermentation titer evaluation method of Example 1-3. After completion of the culture, the production amounts of XMP were measured using HPLC, and the results are shown in Table 8 below.

**[Table 8]**

| | Name of strain | XMP concentration (g/L) |
|---|---|---|
| 1 | KCCM12248P | 13.3 |
| 2 | **KCCM12248P::xmpE(I259S)** | **15.2** |
| 3 | **KCCM12248P::xmpE(I259T)** | **15.5** |
| 4 | **KCCM12248P::xmpE(I259F)** | **13.6** |
| 5 | **KCCM12248P::xmpE(I259P)** | **13.4** |
| 6 | **KCCM12248P::xmpE(I259M)** | **14.2** |
| 7 | **KCCM12248P::xmpE(I259Q)** | **13.5** |
| 8 | **KCCM12248P::xmpE(I259N)** | **13.4** |

As shown in Table 8, it was confirmed that the *Corynebacterium stationis* strains, each in which the amino acid at position 259 from the N-terminus of the MFS transporter was substituted with serine, threonine, phenylalanine, proline, methionine, glutamine, or asparagine, had 0.7% to 16% or more increase in the XMP production ability, as compared to the parent strain, KCCM12248P.

### Example 5: Vector for introducing mutation at V310 position of MFS transporter and Verification of XMP production ability of MFS transporter mutation-introduced Corynebacterium stationis

### 5-1. Construction of vector for introducing MFS transporter mutation

In Example 2-3, it was confirmed that the XMP production ability may be increased when the amino acid at position 310 from the N-terminus of the MFS transporter is mutated. In order to construct a vector for substituting the amino acid at position 310 with another amino acid, site-directed mutagenesis was performed using the pDCM2-xmpE(V310A) vector constructed in Example 2-1 as a template.

In detail, site-directed PCR was performed using a pair of primers of SEQ ID NO: 211 and SEQ ID NO: 212 for introducing V310F mutation into the gene encoding the MFS transporter, a pair of primers of SEQ ID NO: 213 and SEQ ID NO: 214 for introducing V310C mutation, a pair of primers of SEQ ID NO: 215 and SEQ ID NO: 216 for introducing V310L mutation, a pair of primers of SEQ ID NO: 217 and SEQ ID NO: 218 for introducing V310H mutation, a pair of primers of SEQ ID NO: 219 and SEQ ID NO: 220 for introducing V310R mutation, a pair of primers of SEQ ID NO: 221 and SEQ ID NO: 222 for introducing V310I mutation, a pair of primers of SEQ ID NO: 223 and SEQ ID NO: 224 for introducing V310N mutation, a pair of primers of SEQ ID NO: 225 and SEQ ID NO: 226 for introducing V310D mutation, a pair of primers of SEQ ID NO: 227 and SEQ ID NO: 228 for introducing V310G mutation, a pair of primers of SEQ ID NO: 229 and SEQ ID NO: 230 for introducing V310P mutation, a pair of primers of SEQ ID NO: 231 and SEQ ID NO: 232 for introducing V310E mutation, a pair of primers of SEQ ID NO: 233 and SEQ ID NO: 234 for introducing V310M mutation, a pair of primers of SEQ ID NO: 235 and SEQ ID NO: 236 for introducing V310W mutation, a pair of primers of SEQ ID NO: 237 and SEQ ID NO: 238 for introducing V310S mutation, a pair of primers of SEQ ID NO: 239 and SEQ ID NO: 240 for introducing V310T mutation, a pair of primers of SEQ ID NO: 241 and SEQ ID NO: 242 for introducing V310Q mutation, a pair of primers of SEQ ID NO: 243 and SEQ ID NO: 244 for introducing V310K mutation, and a pair of primers of SEQ ID NO: 245 and SEQ ID NO: 246 for introducing V310Y mutation, respectively.

The primer sequences used here are shown in Table 9 below.

**[Table 9]**

| SEQ ID NO. | Sequence name | Sequence (5'->3') |
|---|---|---|
| 211 | pDCM2-xmpE(V310F) | CAACTGGCATGTCGTTTTCGCTGGCGTGCTAGC |
| 212 | pDCM2-xmpE(V310F) | GCTAGCACGCCAGCGAAAACGACATGCCAGTTG |
| 213 | pDCM2-xmpE(V310C) | CAACTGGCATGTCGTTTGCGCTGGCGTGCTAGC |
| 214 | pDCM2-xmpE(V310C) | GCTAGCACGCCAGCGCAAACGACATGCCAGTTG |
| 215 | pDCM2-xmpE(V310L) | CAACTGGCATGTCGTTCTGGCTGGCGTGCTAGC |
| 216 | pDCM2-xmpE(V310L) | GCTAGCACGCCAGCCAGAACGACATGCCAGTTG |
| 217 | pDCM2-xmpE(V310H) | CAACTGGCATGTCGTTCACGCTGGCGTGCTAGC |
| 218 | pDCM2-xmpE(V310H) | GCTAGCACGCCAGCGTGAACGACATGCCAGTTG |
| 219 | pDCM2-xmpE(V310R) | CAACTGGCATGTCGTTAGAGCTGGCGTGCTAGC |
| 220 | pDCM2-xmpE(V310R) | GCTAGCACGCCAGCTCTAACGACATGCCAGTTG |
| 221 | pDCM2-xmpE(V310I) | CAACTGGCATGTCGTTATCGCTGGCGTGCTAGC |
| 222 | pDCM2-xmpE(V310I) | GCTAGCACGCCAGCGATAACGACATGCCAGTTG |
| 223 | pDCM2-xmpE(V310N) | CAACTGGCATGTCGTTAACGCTGGCGTGCTAGC |
| 224 | pDCM2-xmpE(V310N) | GCTAGCACGCCAGCGTTAACGACATGCCAGTTG |
| 225 | pDCM2-xmpE(V310D) | CAACTGGCATGTCGTTGATGCTGGCGTGCTAGC |
| 226 | pDCM2-xmpE(V310D) | GCTAGCACGCCAGCATCAACGACATGCCAGTTG |
| 227 | pDCM2-xmpE(V310G) | CAACTGGCATGTCGTTGGCGCTGGCGTGCTAGC |
| 228 | pDCM2-xmpE(V310G) | GCTAGCACGCCAGCGCCAACGACATGCCAGTTG |
| 229 | pDCM2-xmpE(V310P) | CAACTGGCATGTCGTTCCAGCTGGCGTGCTAGC |
| 230 | pDCM2-xmpE(V310P) | GCTAGCACGCCAGCTGGAACGACATGCCAGTTG |
| 231 | pDCM2-xmpE(V310E) | CAACTGGCATGTCGTTGAAGCTGGCGTGCTAGC |
| 232 | pDCM2-xmpE(V310E) | GCTAGCACGCCAGCTTCAACGACATGCCAGTTG |
| 233 | pDCM2-xmpE(V310M) | CAACTGGCATGTCGTTATGGCTGGCGTGCTAGC |
| 234 | pDCM2-xmpE(V310M) | GCTAGCACGCCAGCCATAACGACATGCCAGTTG |
| 235 | pDCM2-xmpE(V310W) | CAACTGGCATGTCGTTTGGGCTGGCGTGCTAGC |
| 236 | pDCM2-xmpE(V310W) | GCTAGCACGCCAGCCCAAACGACATGCCAGTTG |
| 237 | pDCM2-xmpE(V310S) | CAACTGGCATGTCGTTTCCGCTGGCGTGCTAGC |
| 238 | pDCM2-xmpE(V310S) | GCTAGCACGCCAGCGGAAACGACATGCCAGTTG |
| 239 | pDCM2-xmpE(V310T) | CAACTGGCATGTCGTTACCGCTGGCGTGCTAGC |
| 240 | pDCM2-xmpE(V310T) | GCTAGCACGCCAGCGGTAACGACATGCCAGTTG |
| 241 | pDCM2-xmpE(V310Q) | CAACTGGCATGTCGTTCAGGCTGGCGTGCTAGC |
| 242 | pDCM2-xmpE(V310Q) | GCTAGCACGCCAGCCTGAACGACATGCCAGTTG |
| 243 | pDCM2-xmpE(V310K) | CAACTGGCATGTCGTTAAGGCTGGCGTGCTAGC |
| 244 | pDCM2-xmpE(V310K) | GCTAGCACGCCAGCCTTAACGACATGCCAGTTG |
| 245 | pDCM2-xmpE(V310Y) | CAACTGGCATGTCGTTTACGCTGGCGTGCTAGC |
| 246 | pDCM2-xmpE(V310Y) | GCTAGCACGCCAGCGTAAACGACATGCCAGTTG |

At this time, Solg^{™} Pfu-X DNA polymerase was used as polymerase, and PCR amplification was performed under conditions of denaturation at 95°C for 5 minutes, followed by 24 cycles of denaturation at 95°C for 30 seconds, annealing at 58°C for 30 seconds, and polymerization at 72°C for 10 minutes, and polymerization at 72°C for 5 minutes.

The PCR product amplified above was treated with Dpnl restriction enzyme to remove pDCM2-xmpE(V310) vector which was used as the template, and then transformed into *E*. *coli* DH5a to obtain a vector in which the amino acid at position 310 of the MFS transporter was modified with the desired amino acid. 18 vectors obtained above were named pDCM2-xmpE(V310F), pDCM2-xmpE(V310C), pDCM2-xmpE(V310L), pDCM2-xmpE(V310H), pDCM2-xmpE(V310R), pDCM2-xmpE(V310I), pDCM2-xmpE(V310N), pDCM2-xmpE(V310D), pDCM2-xmpE(V310G), pDCM2-xmpE(V310P), pDCM2-xmpE(V310E), pDCM2-xmpE(V310M), pDCM2-xmpE(V310W), pDCM2-xmpE(V310S), pDCM2-xmpE(V310T), pDCM2-xmpE(V310Q), pDCM2-xmpE(V310K), and pDCM2-xmpE(V310Y), respectively.

### 5-2. Preparation of MFS transporter mutation-introduced Corynebacterium stationis

18 vectors constructed in Example 5-1 were respectively transformed into *Corynebacterium stationis* KCCM12248P strain by electroporation to obtain each strain in which each vector was inserted into the chromosome by recombination of homologous sequence. Each strain was selected on a medium containing 25 mg/L of kanamycin. Each selected primary strain was again subjected to secondary cross-over, and strains were selected, in which the amino acid at position 310 of the MFS transporter was modified with the desired amino acid. The corresponding genetic manipulation was identified through PCR using a pair of primers of SEQ ID NO: 141 and SEQ ID NO: 142 and sequencing analysis using a pair of primers of SEQ ID NO: 143 and SEQ ID NO: 144.

18 strains obtained above were named KCCM12248P::xmpE(V310F), KCCM12248P::xmpE(V310C), KCCM12248P::xmpE(V310L), KCCM12248P::xmpE(V310H), KCCM12248P::xmpE(V310R), KCCM12248P::xmpE(V310I), KCCM12248P::xmpE(V310N), KCCM12248P::xmpE(V310D), KCCM12248P::xmpE(V310G), KCCM12248P::xmpE(V310P), KCCM12248P::xmpE(V310E), KCCM12248P::xmpE(V310M), KCCM12248P::xmpE(V310W), KCCM12248P::xmpE(V310S), KCCM12248P::xmpE(V310T), KCCM12248P::xmpE(V310Q), KCCM12248P::xmpE(V310K), and KCCM12248P::xmpE(V310Y), respectively.

### 5-3. Verification of XMP production ability of MFS transporter mutation-introduced Corynebacterium stationis

18 strains prepared in Example 5-2, KCCM12248P::xmpE(V310A) strain prepared in Example 2-2, and control KCCM12248P strain were cultured by the fermentation titer evaluation method of Example 1-3. After completion of the culture, the production amounts of XMP were measured using HPLC, and the results are shown in Table 10 below.

**[Table 10]**

| | Name of strain | XMP concentration (g/L) |
|---|---|---|
| 1 | KCCM12248P | 13.6 |
| 2 | **KCCM12248P::xmpE(V310A)** | **14.0** |
| 3 | **KCCM12248P::xmpE(V310F)** | **14.2** |
| 4 | **KCCM12248P::xmpE(V310C)** | **14.5** |
| 5 | **KCCM12248P::xmpE(V310I)** | **14.1** |
| 6 | **KCCM12248P::xmpE(V310G)** | **14.9** |
| 7 | **KCCM12248P::xmpE(V310M)** | **15.0** |
| 8 | **KCCM12248P::xmpE(V310W)** | **13.9** |
| 9 | **KCCM12248P::xmpE(V310S)** | **14.1** |
| 10 | **KCCM12248P::xmpE(V310T)** | **14.4** |

As shown in Table 10, it was confirmed that the *Corynebacterium stationis* strains, each in which the amino acid at position 310 from the N-terminus of the MFS transporter was substituted with alanine, phenylalanine, cysteine, isoleucine, glycine, methionine, tryptophan, serine, or threonine, had 2% to 10% or more increase in the XMP production ability, as compared to the parent strain, KCCM12248P.

### Example 6: Vector for introducing mutations at homologous position of MFS transporter variant and Verification of XMP production ability of MFS transporter variant mutation-introduced Corynebacterium stationis

### 6-1. Construction of vector for introducing MFS transporter variant

Plasmids were constructed to introduce I238T and F354S mutations, which exhibit excellent XMP production ability when introduced into *Corynebacterium stationis,* into the homologous positions in the MFS transporters derived from *Corynebacterium glutamicum* ATCC13032 and *Corynebacterium glutamicum* ATCC13869, respectively.

The positions in *Corynebacterium glutamicum* ATCC13032 and *Corynebacterium glutamicum* ATCC13868, which are homologous to positions I238 and F354 of *Corynebacterium stationis,* are shown in Table 11 below.

**[Table 11]**

| Name of strain | *Corynebacterium stationis* | *Corynebacterium glutamicum* ATCC13032 | *Corynebacterium glutamicum* ATCC13869 |
|---|---|---|---|
| Homologous position | F354 | F347 | F332 |
| | 1238 | 1231 | 1216 |

First, vectors for deleting the gene encoding the endogenous MFS transporter of *Corynebacterium stationis* and introducing the gene encoding the MFS transporter derived from *Corynebacterium glutamicum* ATCC13032 and *Corynebacterium glutamicum* ATCC13868 at the corresponding position were constructed, respectively.

In detail, to construct a vector for introducing the gene encoding the MFS transporter derived from *Corynebacterium glutamicum* ATCC13032, PCR was performed using the genomic DNA of the wild-type *Corynebacterium stationis* ATCC6872 as a template and a pair of primers of SEQ ID NO: 247 and SEQ ID NO: 248 and a pair of primers of SEQ ID NO: 251 and SEQ ID NO: 252. To obtain the MFS transporter derived from *Corynebacterium glutamicum* ATCC13032, PCR was performed using the genomic DNA of the *Corynebacterium stationis* ATCC13032 as a template and a pair of primers of SEQ ID NO: 249 and SEQ ID NO: 250.

To construct a vector for introducing the gene encoding the MFS transporter derived from *Corynebacterium glutamicum* ATCC13869, PCR was performed using the genomic DNA of the wild-type *Corynebacterium stationis* ATCC6872 as a template and a pair of primers of SEQ ID NO: 247 and SEQ ID NO: 253 and a pair of primers of SEQ ID NO: 256 and SEQ ID NO: 252. To obtain the MFS transporter derived from *Corynebacterium glutamicum* ATCC13869, PCR was performed using the genomic DNA of the *Corynebacterium glutamicum* ATCC13869 as a template and a pair of primers of SEQ ID NO: 254 and SEQ ID NO: 255.

The primer sequences used here are shown in Table 12 below.

**[Table 12]**

| SEQ ID NO. | Sequence name | Sequence (5'->3') |
|---|---|---|
| 247 | pDC-xmpE-LA-F | AGCTCGGTACCCGGGGATCCTCGCATCGGGTAGGAT CTGCA |
| 248 | xmpE-LA-(Cgl13032)-R | ccttcttcagctacggacaTGATTCTCCTTACTGCAGTTA |
| 249 | (xmpE-LA)-Cgl13032-F | TAACTGCAGTAAGGAGAATCAtgtccgtagctgaagaagg |
| 250 | Cgl13032-(xmpE-RA)-R | TCCCTTGGCTCAAATATTTActagctaactaaagcgacag |
| 251 | (Cgl13032)-xmpE-RA-F | ctgtcgctttagttagctagTAAATATTTGAGCCAAGGGA |
| 252 | pDC-xmpE-RA-R | TGCATGCCTGCAGGTCGACTATGAATGCCCACAGGA ACAGCA |
| 253 | xmpE-LA-(Cgl13869)-R | AGGTTGGCAAACCATCCCATGATTCTCCTTACTGCAG TTA |
| 254 | (xmpE-LA)-Cgl13869-F | TAACTGCAGTAAGGAGAATCATGGGATGGTTTGCCAA CCT |
| 255 | Cgl138692-(xmpE-RA)-R | TCCCTTGGCTCAAATATTTACTAGCTAACTAAAGCGA CAG |
| 256 | (Cgl13869)-xmpE-RA-F | CTGTCGCTTTAGTTAGCTAGTAAATATTTGAGCCAAG GGA |

At this time, Solg^{™} Pfu-X DNA polymerase was used as polymerase, and PCR amplification was performed under conditions of denaturation at 95°C for 5 minutes, followed by 30 cycles of denaturation at 95°C for 30 seconds, annealing at 58°C for 30 seconds, and polymerization at 72°C for 60 seconds, and polymerization at 72°C for 5 minutes.

The PCR product amplified above, and a vector pDCM2 for chromosome transformation, digested with Xbal restriction enzyme, were cloned using Gibson assembly to obtain each recombinant vector. Cloning was performed by mixing a Gibson assembly reagent and each gene fragment in the calculated molar quantity and storing the mixture at 50°C for 1 hour. The recombinant vectors thus constructed were named pDCM2-△xmpE(C.st)_MFS transporter(C.gl ATCC13032) and pDCM2-△xmpE(C.st)_MFS transporter(C.gl ATCC13869) vectors respectively.

### 6-2. Construction of vector for introducing MFS transporter variant mutation

Next, vectors for introducing the mutations of Table 11 into the MFS transporters derived from *Corynebacterium glutamicum* ATCC13032 and *Corynebacterium glutamicum* ATCC13869 were constructed, respectively.

First, a vector for introducing I231T or F347S mutation into the MFS transporter derived from *Corynebacterium glutamicum* ATCC13032 was constructed by site-directed mutagenesis.

In detail, site-directed PCR was performed using the pDCM2-△xmpE(C.st)_MFS transporter(C.gl ATCC13032) vector constructed in Example 6-1 as a template and a pair of primers of SEQ ID NO: 257 and SEQ ID NO: 258 for introducing MFS transporter(I231T) mutation and a pair of primers of SEQ ID NO: 259 and SEQ ID NO: 260 for introducing MFS transporter(F347S) mutation, respectively.

Next, a plasmid for introducing I216T or F332S mutation into the MFS transporter derived from *Corynebacterium glutamicum* ATCC13869 was constructed.

In detail, site-directed PCR was performed using the pDCM2-△xmpE(C.st)_MFS transporter(C.gl ATCC13869) vector constructed in Example 2 as a template and a pair of primers of SEQ ID NO: 257 and SEQ ID NO: 258 for introducing MFS transporter(I216T) mutation and a pair of primers of SEQ ID NO: 259 and SEQ ID NO: 260 for introducing MFS transporter(F332S) mutation, respectively.

The primer sequences used here are shown in Table 13 below.

**[Table 13]**

| SEQ ID NO. | Sequence name | Sequence (5'->3') |
|---|---|---|
| 257 | C. gl 13032 1231T/C. gl 13869 1216T | gcttactctggcgtcaccgcctacatcaacgcattt |
| 258 | C. gl 13032 1231T/C. gl 13869 1216T | aaatgcgttgatgtaggcqagtgacgccagagtaagc |
| 259 | C. gl 13032 F347S/C. gl 13869 F332S | gtttgtggacttaggttccggctttggacctatt |
| 260 | C. gl 13032 F347S/C. gl 13869 F332S | aataggtccaaagccggaacctaagtccacaaac |

At this time, Solg^{™} Pfu-X DNA polymerase was used as polymerase, and PCR amplification was performed under conditions of denaturation at 95°C for 5 minutes, followed by 24 cycles of denaturation at 95°C for 30 seconds, annealing at 58°C for 30 seconds, and polymerization at 72°C for 10 minutes, and polymerization at 72°C for 5 minutes.

The PCR product amplified above was treated with Dpnl restriction enzyme to remove the pDCM2-△xmpE(C.st)_MFS transporter(C.gl ATCC13032) or pDCM2-△xmpE(C.st)_MFS transporter(C.gl ATCC13869) vector used as the template, and then transformed into *E.coli* DH5a strain, respectively to obtain each plasmid in which the amino acid at the specific position of the MFS transporter was mutated. The recombinant plasmids thus obtained were named pDCM2-△xmpE(C.st)_MFS transporter(C.gl ATCC13032, I231T), pDCM2-△xmpE(C.st)_MFS transporter(C.gl ATCC13032, F347S), pDCM2-△xmpE(C.st)_MFS transporter(C.gl ATCC13869, I216T), and pDCM2-△xmpE(C.st)_MFS transporter(C.gl ATCC13869, F332S) vectors, respectively.

### 6-3. Preparation of MFS transporter mutation-introduced Corynebacterium stationis

6 vectors constructed in Example 6-1 and Example 6-2 were respectively transformed into *Corynebacterium stationis* KCCM12248P by electroporation to obtain each strain in which each vector was inserted into the chromosome by recombination of homologous sequence. Each strain was selected on a medium containing 25 mg/L of kanamycin. Each selected primary strain was again subjected to secondary cross-over, and strains were selected, into which the gene encoding the MFS transporter variant was introduced. The corresponding genetic manipulation was identified through PCR and sequencing analysis using a pair of primers of SEQ ID NO: 143 and SEQ ID NO: 144.

6 strains obtained above, into which the gene encoding the MFS transporter variant was introduced, were named KCCM12248P::ΔxmpE(C.st)_MFS transporter(C.gl ATCC13032), KCCM12248P::△xmpE(C.st)_MFS transporter(C.gl ATCC13869), KCCM12248P::ΔxmpE(C.st)_MFS transporter(C.gl ATCC13032, I231T), KCCM12248P::△xmpE(C.st)_MFS transporter(C.gl ATCC13032, F347S), KCCM12248P::ΔxmpE(C.st)_MFS transporter(C.gl ATCC13869, I216T), and KCCM12248P::ΔxmpE(C.st)_MFS transporter(C.gl ATCC13869, F332S), respectively.

### 6-4. Verification of XMP production ability of MFS transporter mutation-introduced Corynebacterium stationis

6 strains prepared in Example 6-3, into which the gene encoding the MFS transporter variant was introduced, were cultured by the fermentation titer evaluation method of Example 1-3. After completion of the culture, the production amounts of XMP were measured using HPLC, and the results are shown in Table 14 below.

**[Table 14]**

| Name of strain | XMP concentration (g/L) |
|---|---|
| KCCM12248P::ΔxmpE(C.st)_MFS transporter(C.gl ATCC13032) | 5.5 |
| KCCM12248P::ΔxmpE(C.st)_MFS transporter(C.gl ATCC13032, I231T) | 6.5 |
| KCCM12248P::ΔxmpE(C.st)_MFS transporter(C.gl ATCC13032, F347S) | 6.3 |
| KCCM12248P::ΔxmpE(C.st)_MFS transporter(C.gl ATCC13869) | 11.4 |
| KCCM12248P::ΔxmpE(C.st)_MFS transporter(C.gl ATCC13869, I216T) | 12.5 |
| KCCM12248P::ΔxmpE(C.st)_MFS transporter(C.gl ATCC13869, F332S) | 12.8 |

As shown in Table 14, it was confirmed that the variants, in which mutations were introduced into the MFS transporter derived from *Corynebacterium glutamicum* ATCC13032, had 14% to 18% or more increase in the XMP production ability, as compared to the wild-type protein of the MFS transporter derived from *Corynebacterium glutamicum* ATCC13032.

It was also confirmed that the variants, in which mutations were introduced into the MFS transporter derived from *Corynebacterium glutamicum* ATCC13869, had 9% to 12% or more increase in the XMP production ability, as compared to the wild-type protein of the MFS transporter derived from *Corynebacterium glutamicum* ATCC13869.

### Example 7: Verification of 5'-guanosine monophosphate (GMP) production ability of MFS transporter variant mutation-introduced Corynebacterium stationis

GMP production was examined using XMP culture media of KCCM12248P::xmpE(I238T), KCCM12248P::xmpE(I259S), KCCM12248P::xmpE(V310A), KCCM12248P::xmpE(F354S), KCCM12248P::△xmpE(C.st)_MFS transporter(C.gl ATCC13032), and KCCM12248P::△xmpE(C.st)_MFS transporter(C.gl ATCC13869) strains, of which XMP production ability was confirmed in Example 2-3 and Example 6, by the following method (Korean Patent Publication No. 10-2011-0105662).

In detail, each strain was cultured by the method of Example 4, and after completion of the culture, the production amounts of XMP were measured using HPLC. To convert the produced XMP into GMP, the following conversion reaction additives and E. coli-derived XMP aminase were added to the XMP culture medium to allow conversion reaction at 40°C for 2.5 hours. After completion of the reaction, the production amounts of GMP were measured using HPLC, and the results are shown in Table 15 below.

The composition of the conversion reaction additives is as follows.

### <Conversion reaction additives>

1.8 g/L phytic acid, 4.8 g/L magnesium sulfate, 3 ml/L nymeen, 2% xylene, 100 mg/L adenine, 7.7 g/L sodium hydrogen phosphate (Na₂HPO₄), 46 g/L glucose (based on 1 liter of distilled water)

**[Table 15]**

| Name of strain | Concentration (g/L) | | Conversion rate (%) |
|---|---|---|---|
| | XMP | GMP | (GMP production/XMP consumption) |
| KCCM12248P::xmpE(I238T) | 15.1 | 11.3 | 74.8 |
| KCCM12248P::xmpE(1259S) | 15.0 | 11.2 | 74.7 |
| KCCM12248P::xmpE(V310A) | 13.5 | 9.9 | 73.3 |
| KCCM12248P::xmpE(F354S) | 15.9 | 11.8 | 74.2 |
| KCCM12248P::ΔxmpE(C.st)_ MFS transporter(C.gl ATCC13032) | 5.2 | 3.7 | 71.2 |
| KCCM12248P::ΔxmpE(C.st)_MFS transporter(C.gl ATCC13869) | 11.1 | 8.0 | 72.1 |

As shown in Table 15, it was confirmed that GMP was produced in a level of about 71% to about 75%, relative to XMP production, through the conversion reaction from XMP produced by KCCM12248P::xmpE(I238T), KCCM12248P::xmpE(I259S), KCCM12248P::xmpE(V310A), KCCM12248P::xmpE(F354S), KCCM12248P::△xmpE(C.st)_MFStransporter(C.gl ATCC13032), and KCCM12248P::△xmpE(C.st)_MFStransporter(C.gl ATCC13869) strains.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

## Claims

1. A major facilitator superfamily (MFS) transporter variant, wherein, in an amino acid sequence of SEQ ID NO: 1 or in an amino acid sequence having 75% or more sequence identity with SEQ ID NO: 1, any one amino acid selected from the group consisting of amino acids corresponding to positions 185, 238, 259, 283, 310, 354, 378, 383, and 403 of the amino sequence of SEQ ID NO: 1 is substituted with another amino acid.

2. The MFS transporter variant of claim 1, wherein the substitution with another amino acid is any one selected from the following substitutions:
(1) a substitution of the amino acid corresponding to position 259 with an amino acid selected from serine, threonine, phenylalanine, proline, methionine, glutamine, and asparagine;
(2) a substitution of the amino acid corresponding to position 310 with an amino acid selected from alanine, phenylalanine, cysteine, isoleucine, glycine, methionine, tryptophan, serine, and threonine;
(3) a substitution of the amino acid corresponding to position 354 with an amino acid selected from serine, tyrosine, asparagine, and leucine;
(4) a substitution of the amino acid corresponding to position 185 with glutamine;
(5) a substitution of the amino acid corresponding to position 238 with threonine;
(6) a substitution of the amino acid corresponding to position 283 with glycine;
(7) a substitution of the amino acid corresponding to position 378 with alanine;
(8) a substitution of the amino acid corresponding to position 383 with leucine; and
(9) a substitution of the amino acid corresponding to position 403 with alanine.

3. The MFS transporter variant of claim 1, wherein the variant consists of any one of amino acid sequence selected from the group consisting of SEQ ID NOS: 7, 9, 11, 13, 15, 17, 19, 21, 51, 55, 57, 59, 75, 81, 85, 91, 93, 95, 97, 105, 111, 117, 119, 121, 123, and 131.

4. The MFS transporter variant of claim 1, wherein an amino acid corresponding to any one position selected from the group consisting of positions 231 and 347 in an amino acid sequence of SEQ ID NO: 3 is substituted with another amino acid.

5. The MFS transporter variant of claim 4, comprising a substitution selected from a substitution of the amino acid corresponding to positions 231 with threonine and a substitution of the amino acid corresponding to position 347 with serine in the amino acid sequence of SEQ ID NO: 3.

6. The MFS transporter variant of claim 4, wherein the variant consists of an amino acid sequence of SEQ ID NO: 133 or 135.

7. The MFS transporter variant of claim 1, wherein an amino acid corresponding to any one position selected from the group consisting of positions 216 and 332 in an amino acid sequence of SEQ **ID** NO: 5 is substituted with another amino acid.

8. The MFS transporter variant of claim 7, comprising a substitution selected from a substitution of the amino acid corresponding to positions 216 with threonine and a substitution of the amino acid corresponding to position 332 with serine in the amino acid sequence of SEQ **ID** NO: 5.

9. The MFS transporter variant of claim 7, wherein the variant consists of an amino acid sequence of SEQ **ID** NO: 137 or 139.

10. A polynucleotide encoding the MFS transporter variant of any one of claims 1 to 9.

11. A microorganism of the genus *Corynebacterium,* the microorganism comprising any one or more selected from the MFS transporter variant of any one of claims 1 to 9; a polynucleotide encoding the MFS transporter variant; and a vector comprising the polynucleotide.

12. The microorganism of claim 11, wherein the microorganism has a purine nucleotide-producing ability.

13. The microorganism of claim 12, wherein the microorganism has an increased purine nucleotide-producing ability, as compared to a wild-type microorganism of the genus *Corynebacterium.*

14. The microorganism of claim 12, wherein the purine nucleotide is any one or more selected from XMP and GMP.

15. The microorganism of claim 11, wherein the microorganism is *Corynebacterium stationis.*

16. A method of producing a purine nucleotide, the method comprising the step of culturing, in a medium, a microorganism of the genus *Corynebacterium* comprising any one or more selected from the MFS transporter variant of any one of claims 1 to 9; a polynucleotide encoding the MFS transporter variant; and a vector comprising the polynucleotide.

17. The method of claim 16, wherein the method further comprises recovering the purine nucleotide from the cultured medium or microorganism.

18. The method of claim 16, wherein the purine nucleotide is any one or more selected from XMP and GMP.

19. Use of a microorganism of the genus *Corynebacterium,* into which any one or more selected from the variant of any one of claims 1 to 9, a polynucleotide encoding thereof, and a vector comprising the polynucleotide is introduced, in the production of a purine nucleotide.
